# EUROPEAN PATENT APPLICATION

(11) **EP 4 424 682 A1**
(43) Date of publication of application: **04.09.2024**
(21) Application number: 22887092.9
(22) Date of filing: 26.10.2022
(51) Int. Cl.: C07D 413/14, C07D 413/12, C07D 417/12, C07D 417/14, H05B 33/02, H10K 50/00

(54) **AMINE COMPOUND AND ORGANIC ELECTROLUMINESCENT ELEMENT USING SAME**

(30) Priority: 28.10.2021 JP 2021176031
(71) Applicant: Hodogaya Chemical Co., Ltd., Tokyo, 105-0021 (JP)
(72) Inventor: KASE, Kouki, Tokyo 105-0021 (JP); YANG, Byung-Sun, Tokyo 105-0021 (JP); HWANG, Moon-Chan, Tokyo 105-0021 (JP); IZUMIDA, Junichi, Tokyo 105-0021 (JP); HIRAYAMA, Yuta, Tokyo 105-0021 (JP); HAYASHI, Shuichi, Tokyo 105-0021 (JP)
(74) Representative: Forstmeyer, Dietmar
(86) International application number: PCT/JP2022/040030
(87) International publication number: WO 2023/074767

(57) **Abstract**

In order to prevent deterioration inside an organic EL element and significantly improve the light extraction efficiency, an object of the present invention is to provide a compound that absorbs light rays with wavelengths of 400 to 410 nm of sunlight, thereby preventing them from affecting a material inside the element, and has a high refractive index in a wavelength range of 450 to 750 nm. The present invention provides an amine compound having a specific benzazole ring structure having a high refractive index. An organic EL element having excellent light emission efficiency is obtained by using the compound of the present invention as a material constituting a capping layer.

## Description

### Technical Field

The present invention relates to a compound suitable for a self-luminescent electronic element favorably used in various display devices, and particularly relates to a compound suitable for an organic electroluminescent element (hereinafter abbreviated as an "organic EL element"), as well as an organic EL element, an electronic element, and an electronic device using the compound.

### Background Art

Since organic EL elements are self-luminescent elements, they are brighter, have superior display viewability, and can provide a clearer display, compared with liquid crystal elements. For these reasons, active studies have been carried out on organic EL elements.

In 1987, C. W. Tang et al. of Eastman Kodak Company produced a practical organic EL element made of an organic material, by developing an element having a stacked layer structure in which various functions were assigned to different materials. They achieved a high luminance of 1,000 cd/m² or higher at a voltage of 10 V or less by stacking a layer of a fluorescent body capable of transporting electrons and a layer of an organic substance capable of transporting holes, and injecting both charges into the fluorescent body layer to cause the layer to emit light (see Patent Literature 1 and Patent Literature 2, for example).

Many improvements have been heretofore made to organic EL elements to put them to practical use, and there are electroluminescent elements in which various functions of the stacked layer structure are subdivided even further, and an anode, a hole injection layer, a hole transport layer, a light emitting layer, an electron transport layer, an electron injection layer, and a cathode are sequentially provided on a substrate. Among such electroluminescent elements, light emitting elements with a bottom emission structure in which light is emitted from the bottom are achieving high efficiency and durability (see Non-Patent Literature 1, for example).

Recently, light emitting elements with a top emission structure, in which a metal having a high work function is used for an anode and light is emitted from the top, are coming into use. Although light emitting elements with a bottom emission structure, in which light is extracted from the bottom where the pixel circuit is located, have an issue in that the area of the light emitting portion is limited, light emitting elements with a top emission structure are advantageous in that a large light emitting portion can be realized because light is extracted from the top and therefore is not blocked by the pixel circuit. In light emitting elements with a top emission structure, translucent electrodes made of LiF/Al/Ag (see Non-Patent Literature 2, for example), Ca/Mg (see Non-Patent Literature 3, for example), LiF/MgAg, or the like are used for a cathode.

In such light emitting elements, when light is emitted by a light emitting layer and incident on another film, if the light is incident at a certain angle or more, the light is totally reflected at the interface between the light emitting layer and the other film. Thus, the light that can be used has been limited to only a part of the emitted light. Recently, light emitting elements have been proposed in which a "capping layer" with a high refractive index is provided on the outer side of a translucent electrode with a low refractive index, in order to improve the light extraction efficiency (see Non-Patent Literature 2 and Non-Patent Literature 3, for example).

Regarding the effect of the capping layer in light emitting elements with a top emission structure, while a light emitting element using Ir(ppy)₃ as a light emitting material has a current efficiency of 38 cd/A in the case of not having a capping layer, the light emitting element has a current efficiency of 64 cd/A in the case of using a ZnSe film with a thickness of 60 nm as a capping layer, which indicates that the efficiency is improved about 1.7 times. Furthermore, it is indicated that the maximum transmittance of the translucent electrode and the capping layer does not necessarily coincide with the maximum efficiency, and the highest light extraction efficiency depends on interference effects (see Non-Patent Literature 3, for example).

Conventionally, it has been proposed to use a fine metal mask to form a capping layer, but this configuration is problematic in that a metal mask is distorted by heat when used at a high temperature, which degrades the positioning precision. ZnSe mentioned above has a high melting point of 1100°C or higher (see Non-Patent Literature 3, for example) and thus cannot be vapor-deposited at an accurate position using a fine metal mask, which may affect the resulting light emitting element itself. Furthermore, film forming through sputtering also affects the light emitting element. Therefore, a capping layer using an inorganic material as a constituent material cannot be suitably used.

In addition, when tris(8-hydroxyquinoline)aluminum (hereinafter abbreviated as "Alq₃") is used for a capping layer for adjusting the refractive index (see Non-Patent Literature 2, for example), since Alq₃, which is known as an organic EL material commonly used as a green light emitting material or an electron transport material, is poor in absorption at wavelengths around 450 nm, which are used for blue light emitting materials, there is a problem in that both the color purity and the light extraction efficiency are degraded when Alq₃ is used in blue light emitting elements.

Moreover, there also is a problem in that an element that is produced using a conventional capping layer transmits light rays with wavelengths of 400 to 410 nm of sunlight, which affects the materials inside the element, resulting in degradation in the color purity and degradation in the light extraction efficiency.

In order to improve the element properties of an organic EL element, in particular, in order for light rays with wavelengths of 400 to 410 nm of sunlight to be absorbed so as not to affect the materials inside the element and for the light extraction efficiency to be significantly improved, there is a demand for a capping layer material that has a high absorption coefficient and a high refractive index, is stable in a thin film state, and has excellent durability.

### Citation List

### Patent Literature

Patent Literature 1: US 5792557
Patent Literature 2: US 5639914
Patent Literature 3: WO 2014/009310
Patent Literature 4: US 2014/0225100A1

### Non-Patent Literature

Non-Patent Literature 1: Proceedings of the 9th Meeting of the Japan Society of Applied Physics, pp. 55-61 (2001)
Non-Patent Literature 2: Appl. Phys. Let., 78, 544 (2001)
Non-Patent Literature 3: Appl. Phys. Let., 82, 466 (2003)
Non-Patent Literature 4: J. Org. Chem., 71, 1802 (2006)
Non-Patent Literature 5: Chem. Rev. 2016, 116, 12564
Non-Patent Literature 6: Tetrahedron, 58, (2002), 9633
Non-Patent Literature 7: Appl. Phys. Lett., 98, 083302 (2011)

### Summary of Invention

It is an object of the present invention to provide, in order to improve the element properties of an organic EL element, a compound that absorbs, in particular, light rays with wavelengths of 400 to 410 nm of sunlight, thereby preventing them from affecting a material inside the element, and also has a high refractive index in a wavelength range of 450 to 750 nm, and it is another object of the present invention to provide an organic EL element in which deterioration inside the element is prevented and that has significantly improved light extraction efficiency, by using this compound.

A material for a capping layer suitable for an organic EL element is required to have the following physical properties: (1) a high absorption coefficient, (2) a high refractive index, (3) vapor deposition capability, (4) good stability in a thin film state, and (5) a high glass transition point. Also, a compound suitable for the present invention is required to have the following physical properties: (1) is capable of absorbing light of 400 to 410 nm, (2) achieves good light extraction efficiency, (3) does not cause degradation in the color purity, (4) has light transmittability without change over time, and (5) has a long lifespan.

Therefore, to achieve the above-described objects, the inventors of the present invention focused on the fact that arylamine-based materials have excellent stability in a thin film state and excellent durability, and produced organic EL elements using, as materials constituting capping layers, specific materials selected from amine compounds having a specific benzazole ring structure having a high refractive index, the selected materials having a high absorbance at wavelengths of 400 to 410 nm in an absorption spectrum at a concentration of 10⁻⁵ mol/L, and thoroughly evaluated the properties of the elements. As a result, the present invention was accomplished.

That is, according to the present invention, a compound represented by a general formula (a) below and an organic EL element are provided.
1) A compound represented by the general formula (a) below. 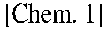 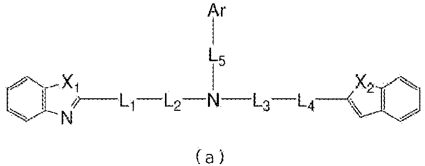
   In the formula above, Ar represents a substituted or unsubstituted aromatic hydrocarbon group, a substituted or unsubstituted aromatic heterocyclic group, or a substituted or unsubstituted fused polycyclic aromatic group;
   L₁ to L₅ may be the same or different, and each represent a single bond, an unsubstituted divalent aromatic hydrocarbon group, an unsubstituted divalent aromatic heterocyclic group, or an unsubstituted divalent fused polycyclic aromatic group; and
   X₁ and X₂ may be the same or different, and each represent an oxygen atom or a sulfur atom.
2) The compound as set forth in clause 1) above, wherein L₁ in the general formula (a) represents an unsubstituted 1,4-phenylene group.
3) The amine compound as set forth in clause 1) above, wherein L₂ to L₅ in the general formula (a) each represent a single bond, an unsubstituted phenylene group, an unsubstituted biphenylene group, or an unsubstituted naphthylene group.
4) The amine compound as set forth in clause 3) above, wherein L₂ to L₅ in the general formula (a) each represent a single bond, an unsubstituted 1,4-phenylene group, an unsubstituted 4,4'-biphenylene group, an unsubstituted 2,6-naphthylene group, or an unsubstituted 2,7-naphthylene group.
5) The amine compound as set forth in clause 1) above, wherein Ar in the general formula (a) represents a substituted or unsubstituted phenyl group, a substituted or unsubstituted biphenyl group, a substituted or unsubstituted naphthyl group, a substituted or unsubstituted phenanthrenyl group, a substituted or unsubstituted pyridyl group, a substituted or unsubstituted quinolyl group, a substituted or unsubstituted benzofuranyl group, a substituted or unsubstituted benzothienyl group, a substituted or unsubstituted dibenzofuranyl group, or a substituted or unsubstituted dibenzothienyl group.
6) The amine compound as set forth in clause 5) above, wherein Ar in the general formula (a) represents an unsubstituted phenyl group, an unsubstituted 4-biphenyl group, an unsubstituted 2-naphthyl group, an unsubstituted 2-phenanthrenyl group, an unsubstituted 3-phenanthrenyl group, an unsubstituted 9-phenanthrenyl group, an unsubstituted 3-pyridyl group, an unsubstituted 3-quinolyl group, an unsubstituted 2-benzofuranyl group, an unsubstituted 2-benzothienyl group, an unsubstituted 2-dibenzofuranyl group, an unsubstituted 3-dibenzofuranyl group, an unsubstituted 2-dibenzothienyl group, or an unsubstituted 3-dibenzothienyl group.
7) An organic EL element including at least an anode electrode, a hole transport layer, a light emitting layer, an electron transport layer, a cathode electrode, and a capping layer arranged in this order, wherein the capping layer contains the amine compound as set forth in any one of clauses 1) to 6) above.
8) The organic EL element as set forth in clause 7) above, wherein the capping layer has an extinction coefficient of 0.2 or more in a wavelength range of 400 to 410 nm, and the amine compound has an absorbance of 0.2 or more in a wavelength range of 400 to 410 nm in an absorption spectrum at a concentration of 10⁻⁵mol/L.
9) The organic EL element as set forth in clause 7) above, wherein the capping layer has a refractive index of 1.85 or more in a wavelength range of 450 to 750 nm.
10) The organic EL element as set forth in clause 7) above, wherein the capping layer is a stack of two or more layers composed of different compounds or a mixed layer containing two or more different compounds, and contains the amine compound as set forth in any one of clauses 1) to 6) above.
11) An electronic element having a pair of electrodes and an organic layer sandwiched therebetween, wherein the organic layer contains the amine compound as set forth in any one of clauses 1) to 6) above.
12) An electronic device including the electronic element as set forth in clause 11) above.

The "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group", or the "substituted or unsubstituted fused polycyclic aromatic group" represented by Ar in the general formula (a) may specifically refer to a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, an imidazopyridyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, a carbolinyl group, or the like, or a group selected from aryl groups having 6 to 30 carbon atoms and heteroaryl groups having 2 to 20 carbon atoms.

Specific examples of the "substituent" in the "substituted aromatic hydrocarbon group", the "substituted aromatic heterocyclic group", or the "substituted fused polycyclic aromatic group" represented by Ar in the general formula (a) include a heavy hydrogen atom; a cyano group and a nitro group; halogen atoms such as a fluorine atom, a chlorine atom, a bromine atom, and an iodine atom; silyl groups such as a trimethylsilyl group and a triphenylsilyl group; linear or branched alkyl groups having 1 to 6 carbon atoms such as a methyl group, an ethyl group, and a propyl group; linear or branched alkyloxy groups having 1 to 6 carbon atoms such as a methyloxy group, an ethyloxy group, and a propyloxy group; alkenyl groups such as a vinyl group and an allyl group; aryloxy groups such as a phenyloxy group and a tolyloxy group; arylalkyloxy groups such as a benzyloxy group and a phenethyloxy group; aromatic hydrocarbon groups or fused polycyclic aromatic groups such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, and a triphenylenyl group; and a pyridyl group, a thienyl group, a furyl group, a pyrrolyl group, a quinolyl group, an isoquinolyl group, a benzofuranyl group, a benzothienyl group, an indolyl group, a carbazolyl group, an imidazopyridyl group, a benzoxazolyl group, a benzothiazolyl group, a quinoxalinyl group, a benzimidazolyl group, a pyrazolyl group, a dibenzofuranyl group, a dibenzothienyl group, a carbolinyl group, and the like; as well as groups selected from aryl groups having 6 to 30 carbon atoms and heteroaryl groups having 2 to 20 carbon atoms; and the like.

These substituents may further be substituted with any of the substituents given as examples above. Furthermore, such a substituent and a benzene ring substituted with the substituent, or a plurality of substituents on the same benzene ring, may be bonded to each other via a single bond, a substituted or unsubstituted methylene group, an oxygen atom, or a sulfur atom to form a ring.

In the compound of the present invention, it is preferable that Ar in the general formula (a) represents a substituted or unsubstituted phenyl group, a substituted or unsubstituted biphenyl group, a substituted or unsubstituted naphthyl group, a substituted or unsubstituted phenanthrenyl group, a substituted or unsubstituted pyridyl group, a substituted or unsubstituted quinolyl group, a substituted or unsubstituted benzofuranyl group, a substituted or unsubstituted benzothienyl group, a substituted or unsubstituted dibenzofuranyl group, a substituted or unsubstituted dibenzothienyl group, a phenanthrenyl group, a benzoxazolyl group, or a benzothiazolyl group.

In the compound of the present invention, it is more preferable that Ar in the general formula (a) represents an unsubstituted phenyl group, an unsubstituted 4-biphenyl group, an unsubstituted 2-naphthyl group, an unsubstituted 2-phenanthrenyl group, an unsubstituted 3-phenanthrenyl group, an unsubstituted 9-phenanthrenyl group, an unsubstituted 3-pyridyl group, an unsubstituted 3-quinolyl group, an unsubstituted 2-benzofuranyl group, an unsubstituted 2-benzothienyl group, an unsubstituted 2-dibenzofuranyl group, an unsubstituted 3-dibenzofuranyl group, an unsubstituted 2-dibenzothienyl group, an unsubstituted 3-dibenzothienyl group, an unsubstituted 1,10-phenanthrolin-2-yl group, an unsubstituted 2-benzoxazolyl group, or an unsubstituted 2-benzothiazolyl group.

Examples of the "divalent aromatic hydrocarbon group", the "divalent aromatic heterocyclic group", or the "divalent fused polycyclic aromatic group" in the "unsubstituted divalent aromatic hydrocarbon group", the "unsubstituted divalent aromatic heterocyclic group", or the "unsubstituted divalent fused polycyclic aromatic group" represented by L₁ to L₅ in the general formula (a) include groups obtained by removing one hydrogen atom from those listed above as examples of the "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group", or the "substituted or unsubstituted fused polycyclic aromatic group" represented by Ar in the general formula (a).

In the compound of the present invention, L₁ in the general formula (a) preferably represents an unsubstituted 1,4-phenylene group.

In the compound of the present invention, L₂ to L₅ in the general formula (a) preferably each represent a single bond, an unsubstituted phenylene group, or an unsubstituted naphthylene group, or preferably each represent a single bond, an unsubstituted phenylene group, an unsubstituted biphenylene group, or an unsubstituted naphthylene group.

In the compound of the present invention, L₂ to L₅ in the general formula (a) more preferably each represent a single bond, an unsubstituted 1,4-phenylene group, an unsubstituted 4,4'-biphenylene group, an unsubstituted 2,6-naphthylene group, or an unsubstituted 2,7-naphthylene group.

In the organic EL element of the present invention, the capping layer preferably has a thickness in a range of 30 to 120 nm, or more preferably in a range of 40 to 80 nm.

In the organic EL element of the present invention, the capping layer preferably has a refractive index of 1.85 or more, or more preferably 1.90 or more, in a wavelength range of 450 to 750 nm.

In the organic EL element of the present invention, the capping layer may be formed using two or more different constituent materials. Specifically, the capping layer may be formed as a stack of two or more layers composed of different compounds, or may be formed as a mixed layer containing two or more different compounds. Note that the capping layer contains at least one amine compound represented by the general formula (a).

The amine compound represented by the general formula (a) of the present invention has the following features: (1) a high absorption coefficient, (2) a high refractive index in a wavelength range of 450 to 750 nm, (3) vapor deposition capability, (4) good stability in a thin film state, and (5) high heat resistance. Therefore, an organic EL element that can achieve significantly improved light extraction efficiency and in which deterioration of materials inside the element is suppressed can be obtained by using the amine compound of the present invention for a capping layer that is provided on the outer side of a transparent or translucent electrode of the organic EL element and has a higher refractive index than the translucent electrode.

### Brief Description of Drawings

[Fig. 1] The structures of Compounds (1) to (12) as examples of a compound of the present invention.
[Fig. 2] The structures of Compounds (13) to (24) as examples of the compound of the present invention.
[Fig. 3] The structures of Compounds (25) to (36) as examples of the compound of the present invention.
[Fig. 4] The structures of Compounds (37) to (48) as examples of the compound of the present invention.
[Fig. 5] The structures of Compounds (49) to (60) as examples of the compound of the present invention.
[Fig. 6] The structures of Compounds (61) to (72) as examples of the compound of the present invention.
[Fig. 7] The structures of Compounds (73) to (84) as examples of the compound of the present invention.
[Fig. 8] The structures of Compounds (85) to (96) as examples of the compound of the present invention.
[Fig. 9] The structures of Compounds (97) to (106) as examples of the compound of the present invention.
[Fig. 10] The structures of Compounds (107) to (116) as examples of the compound of the present invention.
[Fig. 11] The structures of Compounds (117) to (128) as examples of the compound of the present invention.
[Fig. 12] The structures of Compounds (129) to (140) as examples of the compound of the present invention.
[Fig. 13] The structures of Compounds (141) to (155) as examples of the compound of the present invention.
[Fig. 14] The structures of Compounds (156) to (165) as examples of the compound of the present invention.
[Fig. 15] The structures of Compounds (166) to (173) as examples of the compound of the present invention.
[Fig. 16] The structures of Compounds (174) to (184) as examples of the compound of the present invention.
[Fig. 17] The structures of Compounds (185) to (196) as examples of the compound of the present invention.
[Fig. 18] The structures of Compounds (197) to (206) as examples of the compound of the present invention.
[Fig. 19] The structures of Compounds (207) to (209) as examples of the compound of the present invention.
[Fig. 20] An example of the structure of an organic EL element of the present invention.

### Description of Embodiments

Although amine compounds represented by the general formula (a) of the present invention are novel compounds, a benzazole derivative serving as the main backbone of these compounds can be synthesized using a method that is known per se (see Non-Patent Literature 4, for example).

An amine compound represented by the general formula (a) of the present invention can be synthesized by subsequently subjecting the synthesized halogenated benzazole derivative and an arylamine to a coupling reaction with a copper catalyst, a palladium catalyst, or the like. Alternatively, an amine compound represented by the general formula (a) of the present invention can be synthesized similarly by converting the halogenated benzazole derivative to a boronic acid ester derivative and then subjecting the boronic acid ester derivative and a halogenated arylamine to a coupling reaction (see Non-Patent Literature 5 and Non-Patent Literature 6, for example).

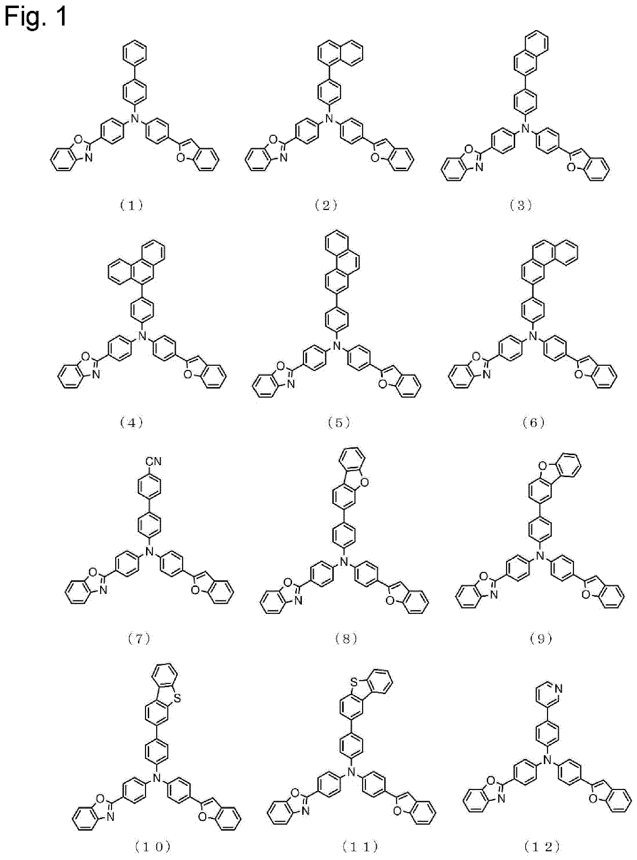

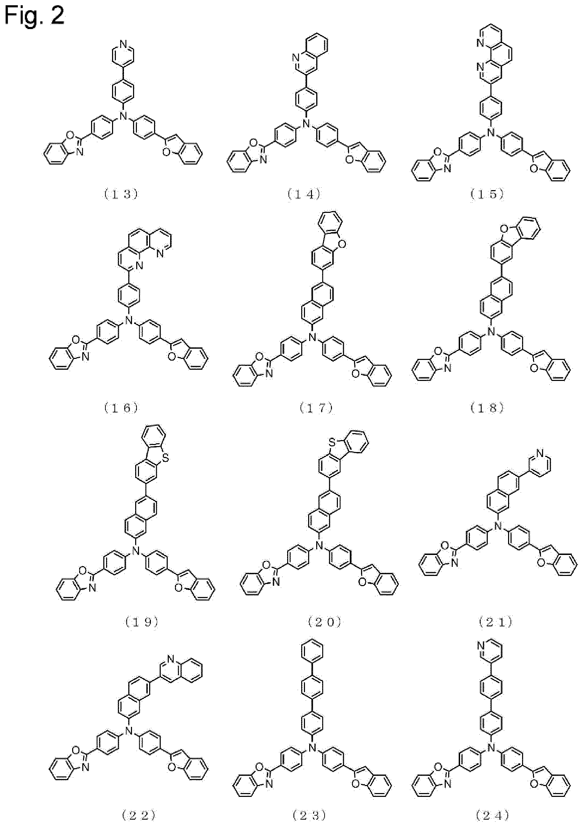

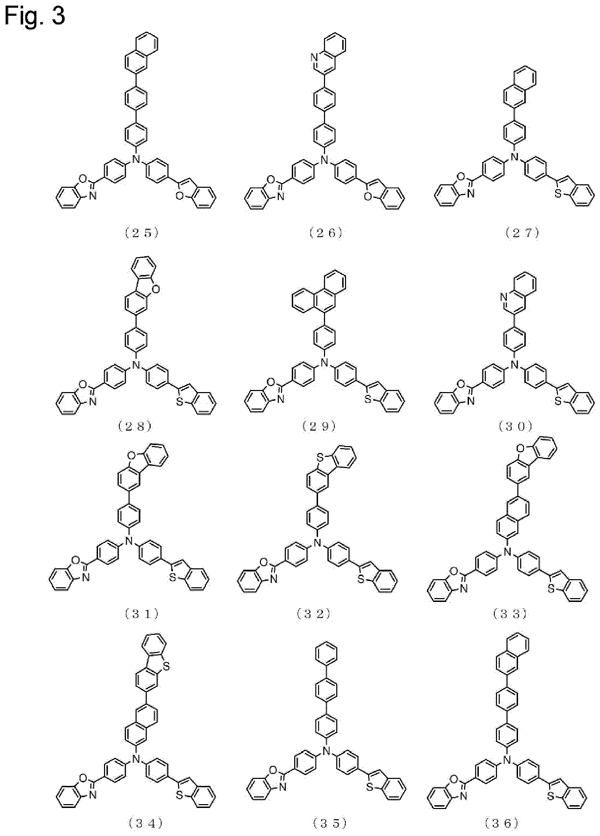

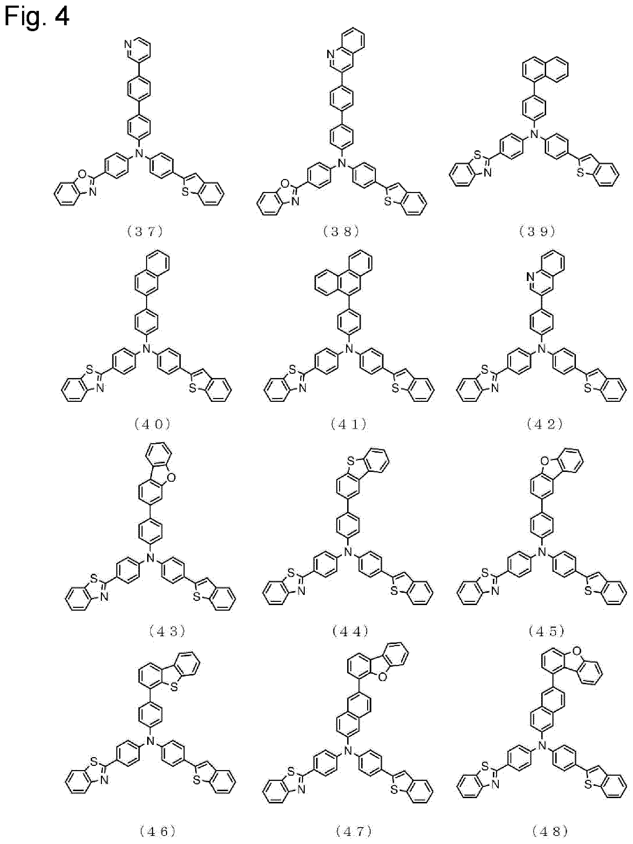

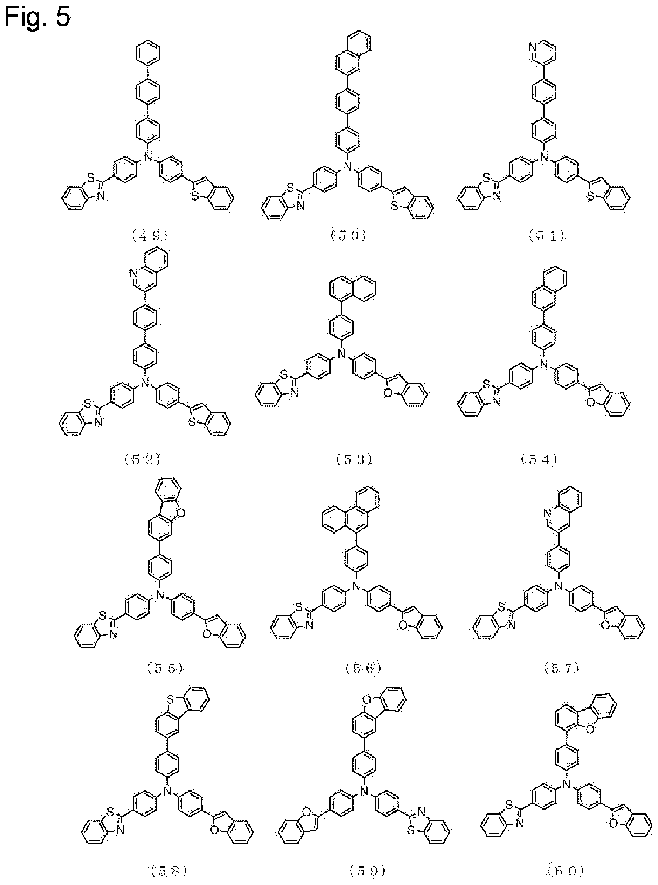

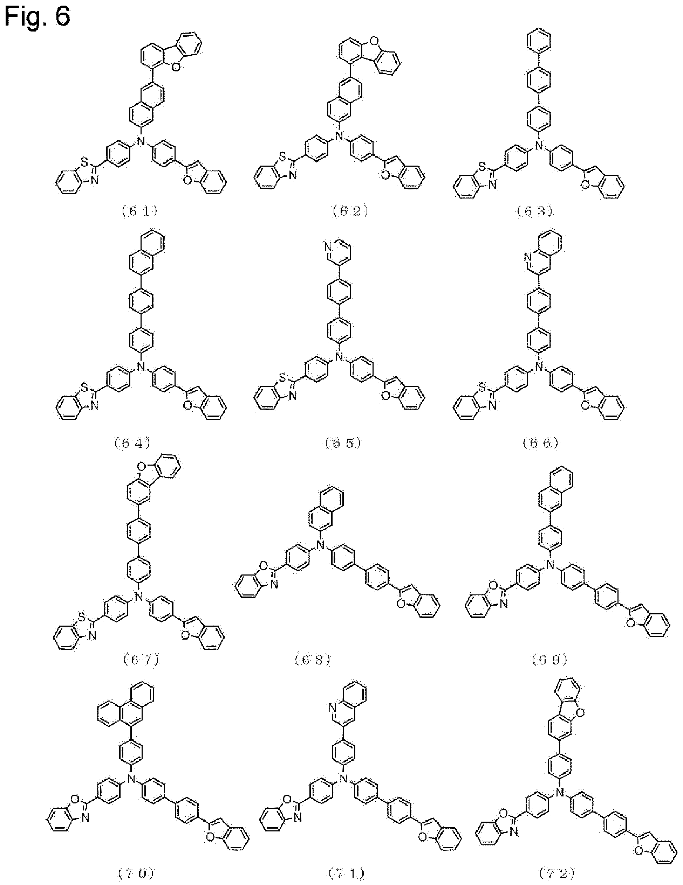

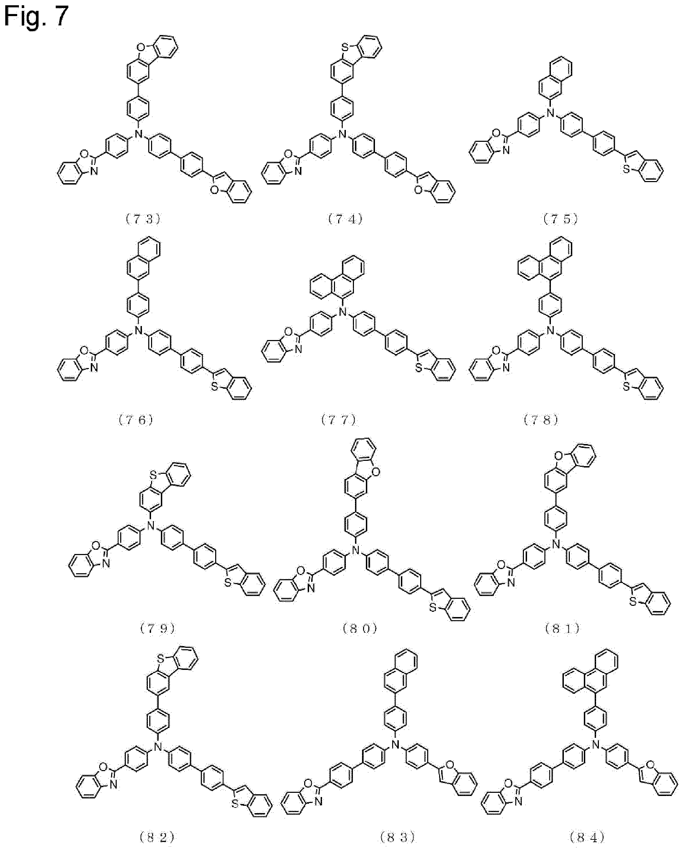

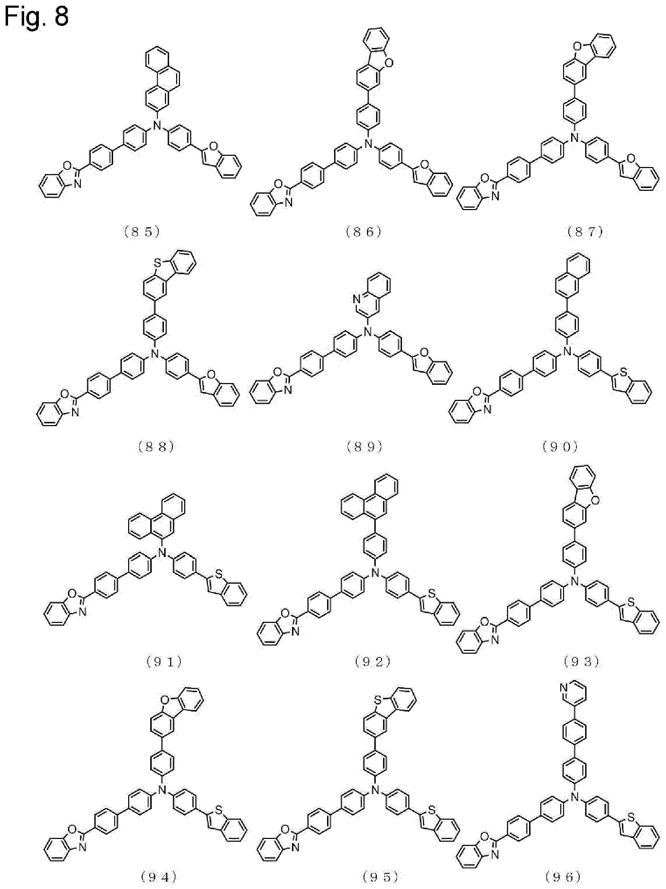

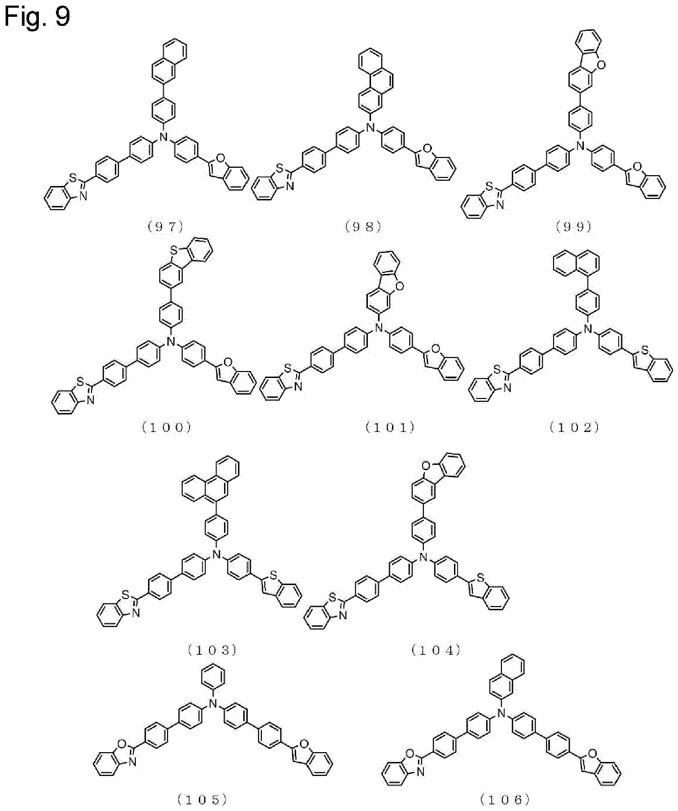

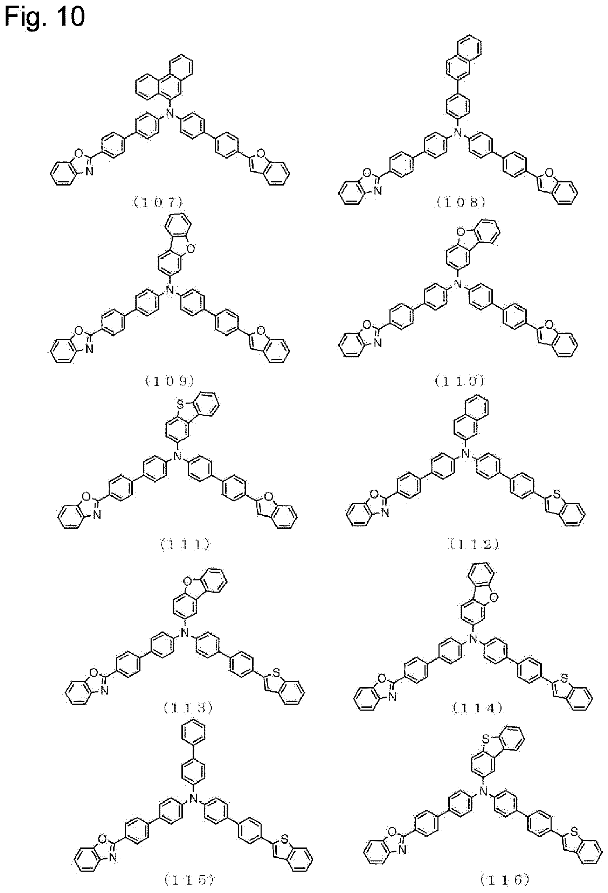

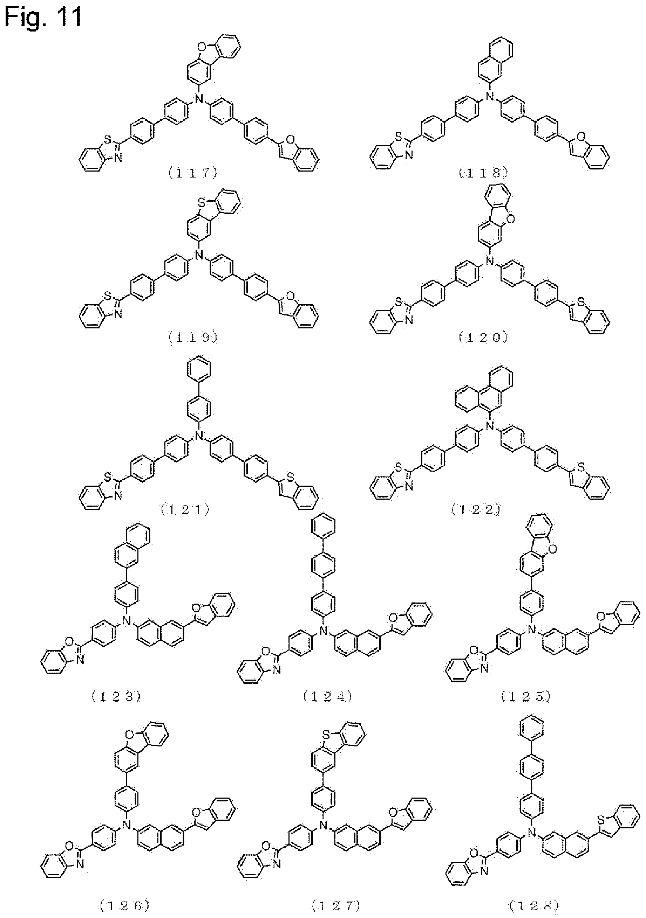

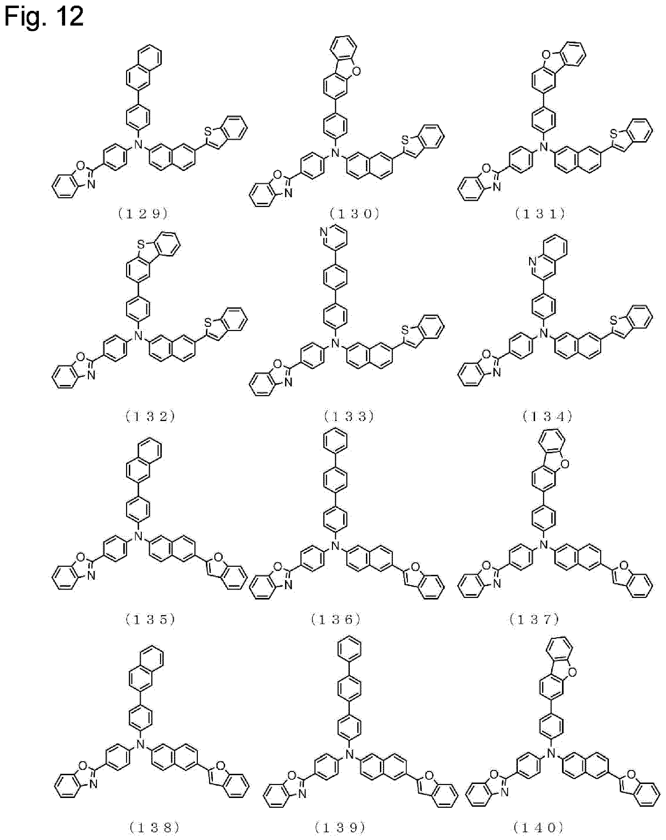

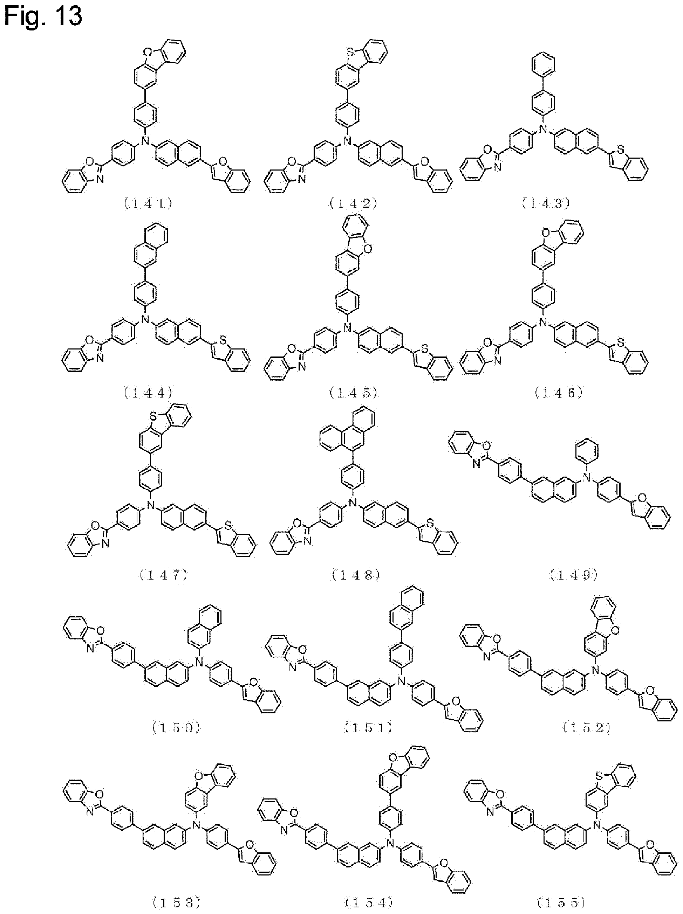

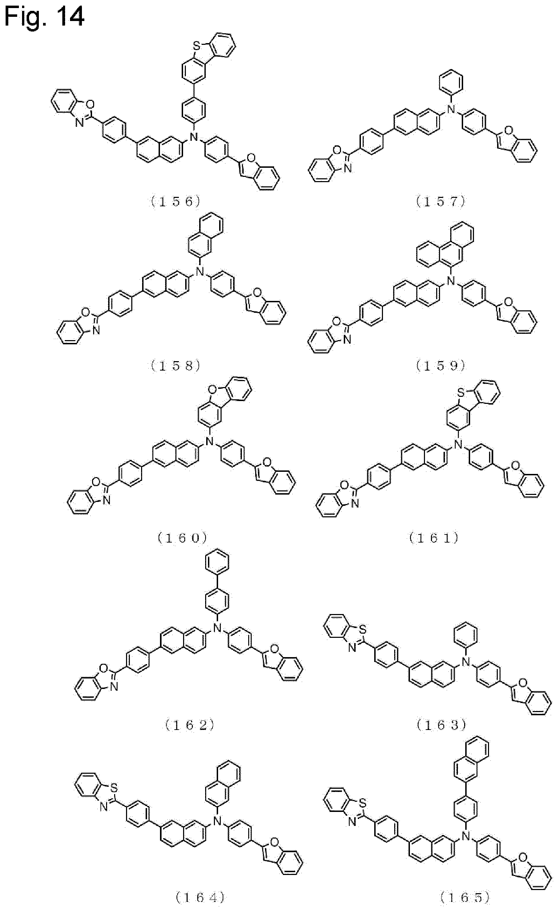

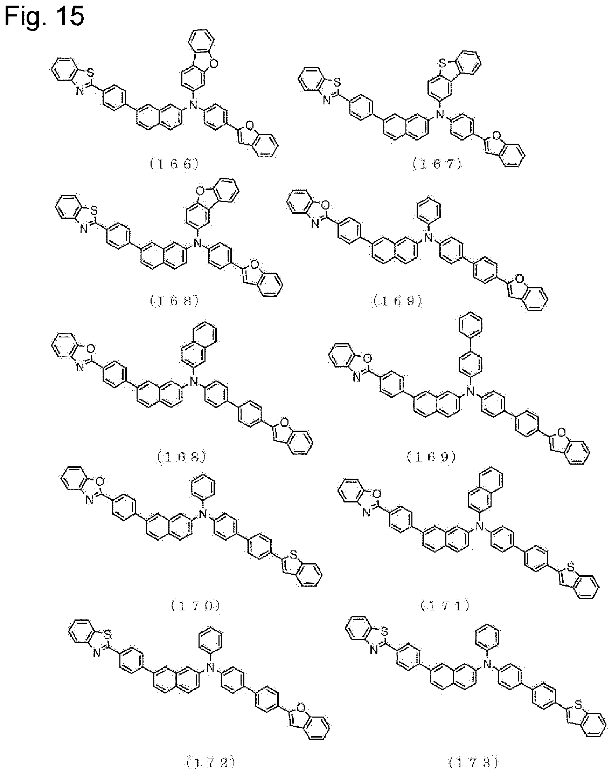

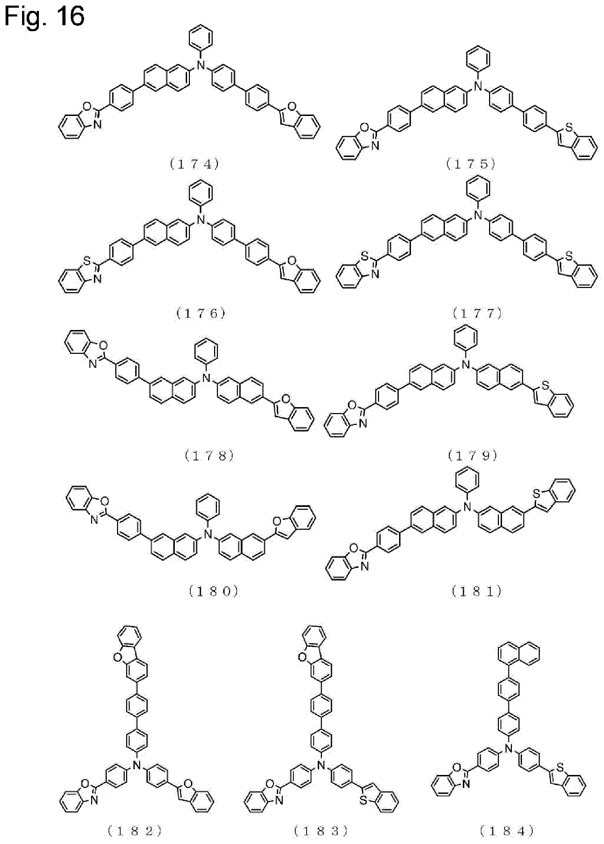

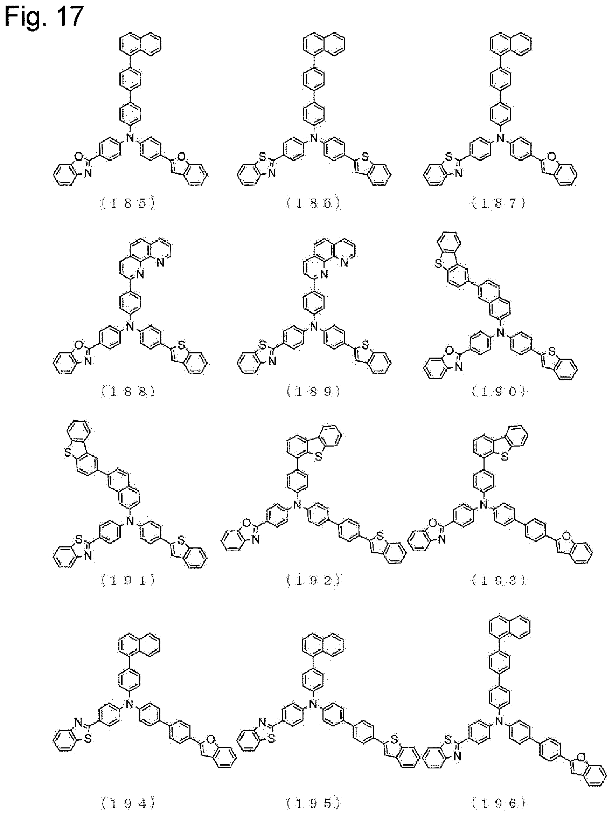

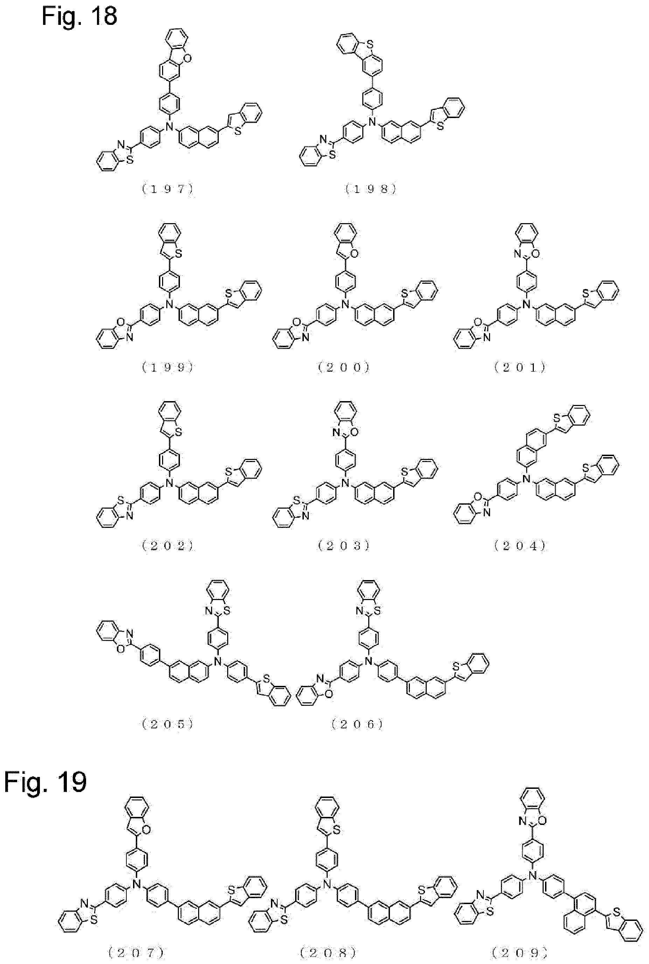

Figs. 1 to 19 show examples of the amine compound represented by the general formula (a) that is suitably used in the organic EL element of the present invention; however, the present invention is not limited to these compounds.

These is no particular limitation on the method for purifying an amine compound represented by the general formula (a), but the amine compound can be purified using a known method used for purification of organic compounds, such as purification by column chromatography, adsorption purification using silica gel, activated carbon, activated clay, or the like, recrystallization or crystallization purification using a solvent, and sublimation purification. In addition, the compound can be identified using NMR analysis or the like. Preferably, the melting point and the glass transition point (Tg), the refractive index and the extinction coefficient, as well as the absorbance and the absorption coefficient are measured as physical properties. The melting point is an indicator of vapor deposition properties, the glass transition point (Tg) is an indicator of stability in a thin film state, the refractive index and the extinction coefficient are indicators related to improvement in the light extraction efficiency, and the absorbance and the absorption coefficient are indicators related to the suppression of deterioration inside the element.

The melting point and the glass transition point (Tg) can be obtained by performing measurement on a compound in power form using a high-sensitivity differential scanning calorimeter (DSC3100SA manufactured by Bruker AXS K.K.).

The refractive index and the extinction coefficient can be obtained by performing measurement on an 80-nm thin film formed on a silicon substrate, using a spectroscopic measurement device (F10-RT-UV manufactured by Filmetrics).

The absorbance can be measured using a solution adjusted to a concentration of 10⁻⁵ mol/L using a toluene solvent, and the absorption coefficient can be measured using solutions adjusted to four different concentrations of 5.0×10⁻⁶ mol/L, 1.0×10⁻⁵ mol/L, 1.5×10⁻⁵ mol/L, and 2.0×10⁻⁵ mol/L using a toluene solvent. In both cases, the measurement can be performed using a UV-NIR spectrophotometer (V-650 manufactured by JASCO Corporation).

The organic EL element of the present invention may have a structure in which, for example, when the organic EL element is for use as a light emitting element with a top emission structure, an anode, a hole transport layer, a light emitting layer, an electron transport layer, a cathode, and a capping layer are sequentially provided on a glass substrate. In addition, the organic EL element may also have a structure in which a hole injection layer is further provided between the anode and the hole transport layer, a structure in which an electron blocking layer is further provided between the hole transport layer and the light emitting layer, a structure in which a hole blocking layer is further provided between the light emitting layer and the electron transport layer, and a structure in which an electron injection layer is further provided between the electron transport layer and the cathode. In these multilayer structures, a single organic layer may have a plurality of functions, and, for example, it is possible to adopt a configuration in which an organic layer serves as both the hole injection layer and the hole transport layer, a configuration in which an organic layer serves as both the hole transport layer and the electron blocking layer, a configuration in which an organic layer serves as both the hole blocking layer and the electron transport layer, a configuration in which an organic layer serves as both the electron transport layer and the electron injection layer, and the like. Furthermore, two or more organic layers having the same function may be stacked, and it is possible to adopt a configuration in which two hole transport layers are stacked, a configuration in which two light emitting layers are stacked, a configuration in which two electron transport layers are stacked, a configuration in which two capping layers are stacked, and the like.

The total film thickness of the layers of the organic EL element is preferably approximately from 200 to 750 nm, and more preferably approximately from 350 to 600 nm. The film thickness of the capping layer is, for example, preferably from 30 to 120 nm, and more preferably from 40 to 80 nm. In this case, it is possible to obtain good light extraction efficiency. Note that the film thickness of the capping layer may be changed as appropriate according to the type of light emitting material used in the light emitting element, the thickness of the organic EL element excluding the capping layer, and the like.

For the anode of the organic EL element of the present invention, an electrode material having a high work function, such as ITO or gold, is used.

For the hole injection layer of the organic EL element of the present invention, it is possible to use arylamine compounds having a molecular structure in which three or more triphenylamine structures are linked to each other via a single bond or a divalent group that does not contain a heteroatom, for example, materials such as starburst triphenylamine derivatives and various triphenylamine tetramers, porphyrin compounds typified by copper phthalocyanine, acceptor type heterocyclic compounds such as hexacyanoazatriphenylene, and coating type polymer materials. The hole injection layer may be formed using one of the above-listed materials alone, or a single layer that is formed using a mixture including another material may be used as the hole injection layer. Alternatively, the hole injection layer may have a stacked layer structure in which layers each formed using one of the above-listed materials alone are stacked, or in which layers each formed using a mixture are stacked, or in which a layer formed using one of the above-listed materials alone and a layer formed using a mixture are stacked. With these materials, a thin film can be formed using vapor deposition, or another known method such as spin coating or inkjet printing.

For the hole transport layer of the organic EL element of the present invention, it is preferable to use benzidine derivatives such as N,N'-diphenyl-N,N'-di(m-tolyl)benzidine (hereinafter abbreviated as "TPD"), N,N'-diphenyl-N,N'-di(α-naphthyl)benzidine, and N,N,N',N'-tetrabiphenylyl benzidine, 1,1-bis[4-(di-4-tolylamino)phenyl]cyclohexane, and, in particular, arylamine compounds having a molecular structure in which two triphenylamine structures are linked to each other via a single bond or a divalent group that does not contain a heteroatom, for example, N,N,N',N'-tetrabiphenylyl benzidine and the like. It is also preferable to use arylamine compounds having a molecular structure in which three or more triphenylamine structures are linked to each other via a single bond or a divalent group that does not contain a heteroatom, for example, various triphenylamine trimers and tetramers and the like. The hole transport layer may be formed using one of the above-listed materials alone, or a single layer that is formed using a mixture including another material may be used as the hole transport layer. Alternatively, the hole transport layer may have a stacked layer structure in which layers each formed using one of the above-listed materials alone are stacked, or in which layers each formed using a mixture are stacked, or in which a layer formed using one of the above-listed materials alone and a layer formed using a mixture are stacked. Furthermore, coating type polymer materials such as poly(3,4-ethylenedioxythiophene)/poly(styrenesulfonate) can be used for the hole injection and transport layers. With these materials, a thin film can be formed using vapor deposition, or another known method such as spin coating or inkjet printing.

Furthermore, a material that is obtained by p-doping a material normally used for the hole injection layer or the hole transport layer with trisbromophenylamine hexachloroantimony, a radialene derivative (see Patent Literature 3, for example), or the like; a polymer compound that has the structure of a benzidine derivative, such as TPD, in a partial structure thereof; and the like can be used for the hole injection layer or the hole transport layer.

For the electron blocking layer of the organic EL element of the present invention, compounds having an electron blocking effect can be used, examples of which include carbazole derivatives such as 4,4',4"-tri(N-carbazolyl)triphenylamine (hereinafter abbreviated as "TCTA"), 9,9-bis[4-(carbazol-9-yl)phenyl]fluorene, 1,3-bis(carbazol-9-yl)benzene (hereinafter abbreviated as "mCP"), and 2,2-bis(4-carbazol-9-yl-phenyl)adamantane; and compounds that have a triphenylsilyl group and a triarylamine structure and are typified by 9-[4-(carbazol-9-yl)phenyl]-9-[4-(triphenylsilyl)phenyl]-9H-fluorene. The electron blocking layer may be formed using one of the above-listed materials alone, or a single layer that is formed using a mixture including another material may be used as the electron blocking layer. Alternatively, the electron blocking layer may have a stacked layer structure in which layers each formed using one of the above-listed materials alone are stacked, or in which layers each formed using a mixture are stacked, or in which a layer formed using one of the above-listed materials alone and a layer formed using a mixture are stacked. With these materials, a thin film can be formed using vapor deposition, or another known method such as spin coating or inkjet printing.

For the light emitting layer of the organic EL element of the present invention, it is possible to use metal complexes of quinolinol derivatives such as Alq₃, various other types of metal complexes, an anthracene derivative, a bisstyrylbenzene derivative, a pyrene derivative, an oxazole derivative, a poly(p-phenylene vinylene) derivative, and the like. Moreover, the light emitting layer may also be formed using a host material and a dopant material. As the host material, an anthracene derivative is preferably used. In addition, the above-listed light emitting materials; as well as a heterocyclic compound having an indole ring as a partial structure of a fused ring; a heterocyclic compound having a carbazole ring as a partial structure of a fused ring; a carbazole derivative; a thiazole derivative; a benzimidazole derivative; a polydialkylfluorene derivative; and the like can be used as the host material. As the dopant material, quinacridone, coumarin, rubrene, perylene, and derivatives thereof; a benzopyran derivative; a rhodamine derivative; an aminostyryl derivative; and the like can be used. The light emitting layer may be formed using one of the above-listed materials alone, or a single layer that is formed using a mixture including another material may be used as the light emitting layer. Alternatively, the light emitting layer may have a stacked layer structure in which layers each formed using one of the above-listed materials alone are stacked, or in which layers each formed using a mixture are stacked, or in which a layer formed using one of the above-listed materials alone and a layer formed using a mixture are stacked.

Furthermore, a phosphorescent emitter can also be used as a light emitting material. As the phosphorescent emitter, a phosphorescent emitter of a metal complex of iridium, platinum, or the like can be used. Examples thereof include a green phosphorescent emitter such as Ir(ppy)₃, a blue phosphorescent emitter such as FIrpic or FIr6, and a red phosphorescent emitter such as Btp₂Ir(acac), and as the host material in this case, carbazole derivatives such as 4,4'-di(N-carbazolyl)biphenyl, TCTA, and mCP can be used as host materials having hole injectability and transportability. As the host material that has electron transportability, p-bis(triphenylsilyl)benzene, 2,2',2"-(1,3,5-phenylene)-tris(1-phenyl-1H-benzimidazole), and the like can be used.

When doping the host material with a phosphorescent light emitting material, in order to avoid concentration quenching, it is preferable that the amount of the phosphorescent light emitting material is in a range of 1 to 30 wt% with respect to the entire light emitting layer and the doping is performed through co-deposition.

Furthermore, materials that emit delayed fluorescence, such as CDCB derivatives such as PIC-TRZ, CC2TA, PXZ-TRZ, and 4CzIPN, can also be used as the light emitting material (see Non-Patent Literature 7, for example.) With these materials, a thin film can be formed using vapor deposition, or another known method such as spin coating or inkjet printing.

For the hole blocking layer of the organic EL element of the present invention, compounds having a hole blocking effect can be used, examples of which include a phenanthroline derivative such as bathocuproine, a metal complex of a quinolinol derivative, such as aluminum(III)bis(2-methyl-8-quinolinato)-4-phenylphenolate (hereinafter abbreviated as "BAlq"), various types of rare-earth complexes, a triazole derivative, a triazine derivative, a pyrimidine derivative, an oxadiazole derivative, a benzazole derivative, and the like. These materials may also serve as the materials for the electron transport layer. The hole blocking layer may be formed using one of the above-listed materials alone, or a single layer that is formed using a mixture including another material may be used as the hole blocking layer. Alternatively, the hole blocking layer may have a stacked layer structure in which layers each formed using one of the above-listed materials alone are stacked, or in which layers each formed using a mixture are stacked, or in which a layer formed using one of the above-listed materials alone and a layer formed using a mixture are stacked. With these materials, a thin film can be formed using vapor deposition, or another known method such as spin coating or inkjet printing.

For the electron transport layer of the organic EL element of the present invention, it is possible to use metal complexes of quinolinol derivatives such as Alq₃ and BAlq, various other types of metal complexes, a triazole derivative, a triazine derivative, a pyrimidine derivative, an oxadiazole derivative, a pyridine derivative, a benzimidazole derivative, a benzazole derivative, a thiadiazole derivative, an anthracene derivative, a carbodiimide derivative, a quinoxaline derivative, a pyridoindole derivative, a phenanthroline derivative, a silole derivative, and the like. The electron transport layer may be formed using one of the above-listed materials alone, or a single layer that is formed using a mixture including another material may be used as the electron transport layer. Alternatively, the electron transport layer may have a stacked layer structure in which layers each formed using one of the above-listed materials alone are stacked, or in which layers each formed using a mixture are stacked, or in which a layer formed using one of the above-listed materials alone and a layer formed using a mixture are stacked. With these materials, a thin film can be formed using vapor deposition, or another known method such as spin coating or inkjet printing.

For the electron injection layer of the organic EL element of the present invention, it is possible to use alkali metal salts such as lithium fluoride and cesium fluoride, alkaline earth metal salts such as magnesium fluoride, metal complexes of quinolinol derivatives such as lithium quinolinol, metal oxides such as aluminum oxide, metals such as ytterbium (Yb), samarium (Sm), calcium (Ca), strontium (Sr), and cesium (Cs), and the like. When an electron transport layer and a cathode are suitably selected, the electron injection layer can be omitted.

Furthermore, a material obtained by n-doping a material normally used for the electron injection layer or the electron transport layer with a metal such as cesium can be used for the electron injection layer or the electron transport layer.

For the cathode of the organic EL element of the present invention, an electrode material having a low work function such as aluminum; an alloy having an even lower work function such as a magnesium-silver alloy, a magnesium-calcium alloy, a magnesium-indium alloy, or an aluminum-magnesium alloy; or ITO, IZO, or the like is used as an electrode material.

For the capping layer of the organic EL element of the present invention, it is preferable to use amine compounds represented by the general formula (a). The capping layer may be formed using one of these amine compounds alone, or a single layer that is formed using a mixture including another material may be used as the capping layer. Alternatively, the capping layer may have a stacked layer structure in which layers each formed using one of these amine compounds alone are stacked, or in which layers each formed using a mixture are stacked, or in which a layer formed using one of these amine compounds alone and a layer formed using a mixture are stacked. With these materials, a thin film can be formed using vapor deposition, or another known method such as spin coating or inkjet printing.

In the foregoing description, an organic EL element with a top emission structure has been described, but the present invention is not limited thereto, and can also be applied in a similar manner to an organic EL element with a bottom emission structure or an organic EL element with a dual emission structure, in which light is emitted from both the top and the bottom. In these cases, an electrode located on a side where light is extracted from the light emitting element to the outside has to be transparent or translucent.

The refractive index of the material constituting the capping layer is preferably greater than the refractive index of the electrode located adjacent to the capping layer. More specifically, while the capping layer improves the light extraction efficiency of the organic EL element, this effect is more effective when the reflectance at the interface between the capping layer and a material that is in contact with the capping layer is greater, because the effect of optical interference is greater. Therefore, the refractive index of the material constituting the capping layer is preferably greater than the refractive index of the electrode located adjacent to the capping layer. It is sufficient that the refractive index of the capping layer is 1.70 or more, but the refractive index of the capping layer is more preferably 1.80 or more, or even more preferably 1.85 or more.

Hereinafter, embodiments of the present invention will be described in greater detail using examples, but the present invention is not limited to the examples below and includes other matter that does not depart from the gist of the present invention.

### Examples

### Example 1

### < Synthesis of (4-benzofuran-2-yl-phenyl)-(4-benzothiazol-2-yl-phenyl)-(4-naphthalen-2-yl-phenyl)ami ne: Compound (54)>

30.0 g of 4-benzothiazol-2-yl-phenyl-amine, 19.9 g of 2-(4-bromophenyl)-naphthalene, 0.6 g of tris(dibenzylideneacetone)dipalladium(0), 0.8 g of tri-t-butylphosphine, and 9.6 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through crystallization using a chlorobenzene/acetone mixed solvent to obtain 26.8 g (with a yield of 88.9%) of (4-benzothiazol-2-yl-phenyl)-(4-naphthalen-2-yl-phenyl)amine.

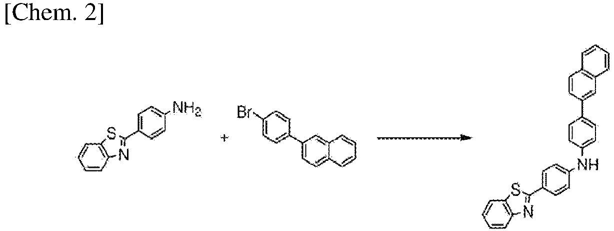

Subsequently, 13.4 g of (4-benzothiazol-2-yl-phenyl)-(4-naphthalen-2-yl-phenyl)-amine, 9.0 g of 2-(4-bromophenyl)-benzofuran, 0.3 g of tris(dibenzylideneacetone)dipalladium(0), 0.1 g of tri-t-butylphosphine, and 4.5 g of sodium t-butoxide were placed in a nitrogen-purged reaction vessel and stirred in a toluene solvent for 3 hours under reflux. After the reaction system was allowed to cool, chlorobenzene was added thereto, the mixture was filtered, and the filtrate was concentrated to obtain a crude product. The obtained crude product was purified through crystallization using a THF/acetone mixed solvent to obtain 11.2 g (with a yield of 57.7%) of a yellow powder of (4-benzofuran-2-yl-phenyl)-(4-benzothiazol-2-yl-phenyl)-(4-naphthalen-2-yl-phenyl)-ami ne: Compound (54).

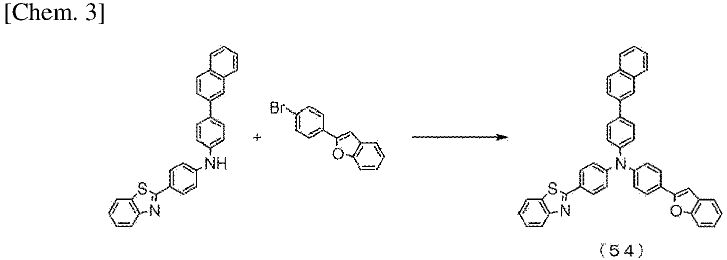

The structure of the obtained yellow powder was identified using NMR.

In ¹H-NMR(DMSO-d₆), the following signals of 28 hydrogens were detected.
δ (ppm) = 8.25 (1H), 8.12 (1H), 8.06 (2H), 8.02 (3H), 7.94 (3H), 7.89 (3H), 7.66 (1H), 7.63 (1H), 7.59-7.50 (3H), 7.44 (1H), 7.38 (1H), 7.35-7.25 (6H), 7.23 (2H).

### Example 2

### <Synthesis of (4'-benzothiophen-2-yl-biphenyl-4-yl)-(4-benzoxazol-2-yl-phenyl)-(4-dibenzofuran-3-yl-phenyl)amine: Compound (80)>

30.0 g of 4-dibenzofuran-3-yl-phenyl-amine, 34.1 g of 2-(4-bromophenyl)-benzoxazole, 2.5 g of tris(dibenzylideneacetone)dipalladium(0), 4.1 g of 2,2'-bis(diphenylphosphino)-1,1'-binaphthyl, and 13.7 g of sodium t-butoxide were placed in a nitrogen-purged reaction vessel and stirred in a toluene solvent for 3 hours under reflux. After the reaction system was allowed to cool, methanol was added thereto, and the precipitated solid was collected through filtration to obtain a crude product. The obtained crude product was purified through recrystallization using a 1,2-dichlorobenzene solvent to obtain 41.5 g (with a yield of 83.8%) of (4-benzoxazol-2-yl-phenyl)-(4-dibenzofuran-3-yl-phenyl)-amine.

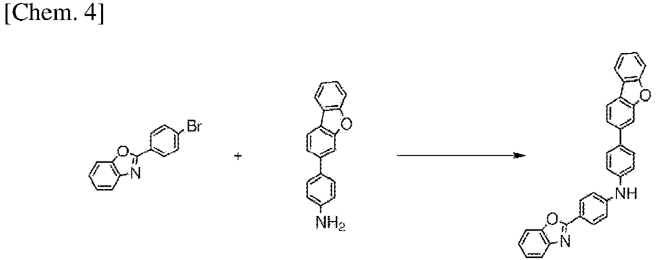

Subsequently, 9.0 g of (4-benzoxazol-2-yl-phenyl)-(4-dibenzofuran-3-yl-phenyl)-amine, 7.0 g of 2-(4'-chloro-biphenyl-4-yl)benzothiophene, 0.4 g of tris(dibenzylideneacetone)dipalladium(0), 0.2 g of tri-t-butylphosphine, and 3.8 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through recrystallization using a chlorobenzene solvent to obtain 8.7 g (with a yield of 59.2%) of a yellow powder of (4'-benzothiophen-2-yl-biphenyl-4-yl)-(4-benzoxazol-2-yl-phenyl)-(4-dibenzofuran-3-yl-phenyl)amine: Compound (80).

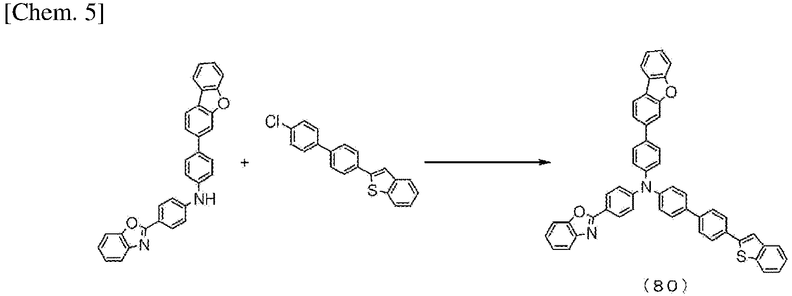

The structure of the obtained yellow powder was identified using NMR.

In ¹H-NMR(DMSO-d₆), the following signals of 32 hydrogens were detected.
δ (ppm) = 8.21 (2H), 8.15 (2H), 8.00 (4H), 7.94-7.68 (12H), 7.55 (1H), 7.49-7.28 (9H), 7.24 (2H).

### Example 3

### < Synthesis of (4-benzofuran-2-yl-phenyl)-(4-benzoxazol-2-yl-phenyl)-(4-dibenzofuran-3-yl-phenyl)ami ne: Compound (8)>

7.5 g of (4-benzoxazol-2-yl-phenyl)-(4-dibenzofuran-3-yl-phenyl)-amine, 4.8 g of 2-(4-bromophenyl)benzofuran, 0.2 g of tris(dibenzylideneacetone)dipalladium(0), 0.1 g of tri-t-butylphosphine, and 2.4 g of sodium t-butoxide were placed in a nitrogen-purged reaction vessel and stirred in a toluene solvent for 2 hours under reflux. The reaction system was allowed to cool and then filtered, and the filtrate was concentrated to obtain a crude product. The obtained crude product was purified through crystallization using a toluene/acetone mixed solvent to obtain 6.6 g (with a yield of 61.7%) of a yellow powder of
(4-benzofuran-2-yl-phenyl)-(4-benzoxazol-2-yl-phenyl)-(4-dibenzofuran-3-yl-phenyl)ami ne: Compound (8).

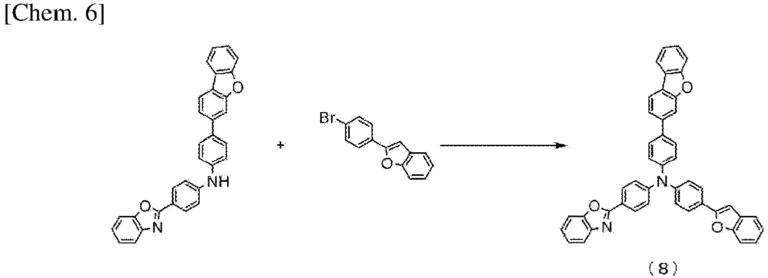

The structure of the obtained yellow powder was identified using NMR.

In ¹H-NMR (CDCl₃), the following signals of 28 hydrogens were detected.
δ (ppm) = 8.15 (2H), 8.00 (1H), 7.97 (1H), 7.83 (2H), 7.80 (1H), 7.75 (1H), 7.67 (2H), 7.59 (4H), 7.52 (1H), 7.47 (1H), 7.39-7.26 (10H), 7.23 (1H), 6.98 (1H).

### Example 4

### < Synthesis of (4-benzofuran-2-yl-phenyl)-(4-benzoxazol-2-yl-phenyl)-(4' -naphthalen-2-yl-biphenyl-4-y l)amine: Compound (25)>

9.0 g of (4-benzoxazol-2-yl-phenyl)-(4-benzofuran-2-yl-phenyl)amine, 8.8 g of 2-(4'-bromo-biphenyl-4-yl)naphthalene, 0.6 g of tris(dibenzylideneacetone)dipalladium(0), 0.4 g of tri-t-butylphosphine, and 3.2 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 14.2 g (with a yield of 93.4%) of a yellow powder of (4-benzofuran-2-yl-phenyl)-(4-benzoxazol-2-yl-phenyl)-(4' -naphthalen-2-yl-biphenyl-4-y l)amine: Compound (25).

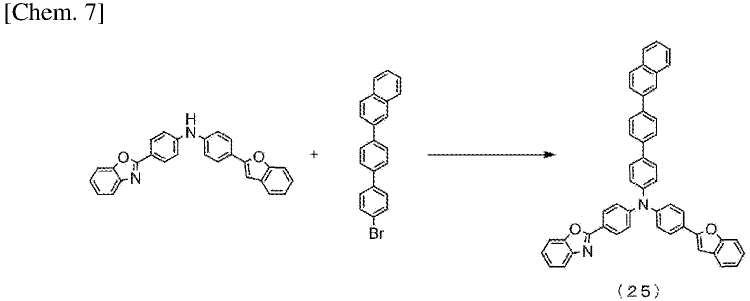

The structure of the obtained yellow powder was identified using NMR.

In ¹H-NMR (CDCl₃), the following signals of 32 hydrogens were detected.
δ (ppm) = 8.15 (2H), 8.10 (1H), 7.94 (1H), 7.92 (1H), 7.88 (1H), 7.82 (4H), 7.80 (1H), 7.75 (1H), 7.73 (2H), 7.65 (2H), 7.58 (1H), 7.56 (1H), 7.51 (3H), 7.33 (2H), 7.31-7.26 (7H), 7.23 (1H), 6.98 (1H).

### Example 5

### < Synthesis of (4-benzothiophen-2-yl-phenyl)-(4-benzoxazol-2-yl-phenyl)-(4-dibenzofuran-3-yl-phenyl) amine: Compound (28)>

7.5 g of (4-benzoxazol-2-yl-phenyl)-(4-dibenzofuran-3-yl-phenyl)-amine, 5.0 g of 2-(4-bromo-phenyl)benzothiophene, 0.2 g of tris(dibenzylideneacetome)dipalladium(0), 0.1 g of tri-t-butylphosphine, and 2.4 g of sodium t-butoxide were placed in a nitrogen-purged reaction vessel and stirred in a toluene solvent for 3 hours under reflux. The reaction system was allowed to cool and then filtered, and the filtrate was concentrated to obtain a crude product. The obtained crude product was purified through crystallization using a toluene/acetone mixed solvent to obtain 5.7 g (with a yield of 51.8%) of a yellow powder of (4-benzothiophen-2-yl-phenyl)-(4-benzoxazol-2-yl-phenyl)-(4-dibenzofuran-3-yl-phenyl) amine: Compound (28).

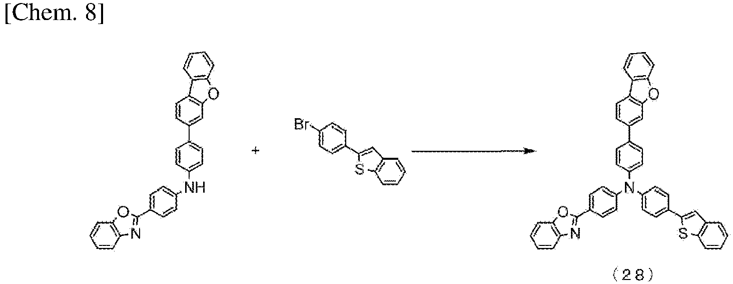

The structure of the obtained yellow powder was identified using NMR.

In ¹H-NMR (CDCl₃), the following signals of 28 hydrogens were detected.
δ (ppm) = 8.15 (2H), 8.00 (1H), 7.97 (1H), 7.83 (1H), 7.80 (1H), 7.76 (2H), 7.67 (4H), 7.59 (3H), 7.51 (1H), 7.47 (1H), 7.39-7.25 (11H).

### Example 6

### < Synthesis of (4-benzothiophen-2-yl-phenyl)-(4-benzoxazol-2-yl-phenyl)-(4'-naphthalen-2-yl-biphenyl-4-yl)amine: Compound (36)>

9.0 g of (4-benzoxazol-2-yl-phenyl)-(4-benzothiophen-2-yl-phenyl)amine, 8.5 g of 2-(4'-bromo-biphenyl-4-yl)naphthalene, 0.6 g of tris(dibenzylideneacetone)dipalladium(0), 0.4 g of tri-t-butylphosphine, and 3.1 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 8.7 g (with a yield of 58.0%) of a yellow powder of (4-benzothiophen-2-yl-phenyl)-(4-benzoxazol-2-yl-phenyl)-(4'-naphthalen-2-yl-biphenyl-4-yl)amine: Compound (36).

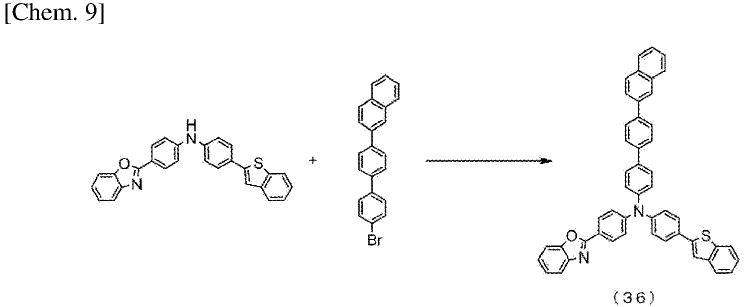

The structure of the obtained yellow powder was identified using NMR.

In ¹H-NMR (CDCl₃), the following signals of 32 hydrogens were detected.
δ (ppm) = 8.15 (2H), 8.10 (1H), 7.94 (1H), 7.91 (1H), 7.88 (1H), 7.84-7.71 (8H), 7.68 (2H), 7.65 (2H), 7.56 (1H), 7.50 (3H), 7.34 (3H), 7.30 (3H), 7.26 (4H).

### Example 7

### < Synthesis of (4-benzothiophen-2-yl-phenyl)-(4-benzothiazol-2-yl-phenyl)-(4-naphthalen-2-yl-phenyl)-amine: Compound (40)>

13.4 g of (4-benzothiazol-2-yl-phenyl)-(4-naphthalen-2-yl-phenyl)-amine, 9.5 g of 2-(4-bromo-phenyl)benzothiophene, 0.3 g of tris(dibenzylideneacetome)dipalladium(0), 0.1 g of tri-t-butylphosphine, and 4.5 g of sodium t-butoxide were placed in a nitrogen-purged reaction vessel and stirred in a toluene solvent for 5 hours under reflux. The reaction system was allowed to cool and then filtered, and the filtrate was concentrated to obtain a crude product. The obtained crude product was purified through crystallization using a THF/acetone mixed solvent to obtain 13.2 g (with a yield of 66.3%) of a yellow powder of (4-benzothiophen-2-yl-phenyl)-(4-benzothiazol-2-yl-phenyl)-(4-naphthalen-2-yl-phenyl)a mine: Compound (40).

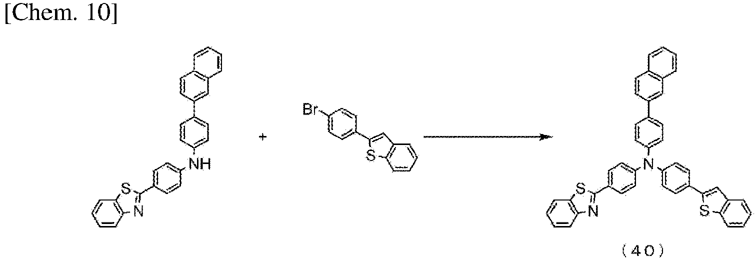

The structure of the obtained yellow powder was identified using NMR.

In ¹H-NMR (CDCl₃), the following signals of 28 hydrogens were detected.
δ (ppm) = 8.05 (1H), 8.04 (1H), 8.00 (2H), 7.91 (2H), 7.88 (2H), 7.82 (1H), 7.76 (2H), 7.70 (2H), 7.67 (2H), 7.51 (1H), 7.49 (3H), 7.36 (2H), 7.31 (3H), 7.26 (2H), 7.24 (2H).

### Example 8

### < Synthesis of (4-benzothiophen-2-yl-phenyl)-(4-benzothiazol-2-yl-phenyl)-(4-dibenzofuran-3-yl-phenyl )amine: Compound (43)>

5.0 g of (4-benzothiazol-2-yl-phenyl)-(4-dibenzofuran-3-yl-phenyl)-amine, 3.4 g of 2-(4-bromo-phenyl)benzothiophene, 0.2 g of tris(dibenzylideneacetome)dipalladium(0), 0.2 g of tri-t-butylphosphine, and 1.5 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through crystallization using a monochlorobenzene/acetone mixed solvent to obtain 5.8 g (with a yield of 78.9%) of a yellow powder of (4-benzothiophen-2-yl-phenyl)-(4-benzothiazol-2-yl-phenyl)-(4-dibenzofuran-3-yl-phenyl )amine: Compound (43).

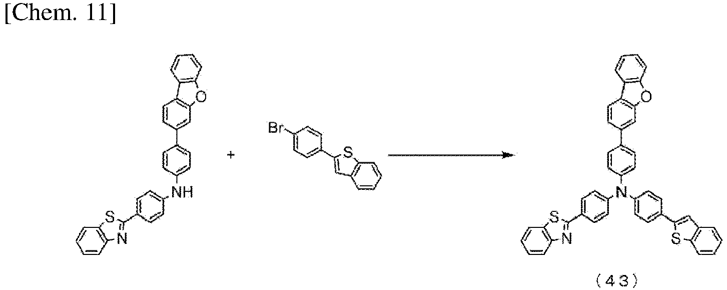

The structure of the obtained yellow powder was identified using NMR.

In ¹H-NMR (CDCl₃), the following signals of 28 hydrogens were detected.
δ (ppm) = 8.04 (1H), 8.00 (3H), 7.97 (1H), 7.89 (1H), 7.83 (1H), 7.80 (1H), 7.77 (1H), 7.67 (4H), 7.60 (2H), 7.51 (1H), 7.48 (2H), 7.40-7.28 (6H), 7.27 (2H), 7.24 (2H).

### Example 9

### < Synthesis of (4-benzothiophen-2-yl-phenyl)-(4-benzothiazol-2-yl-phenyl)-(4-dibenzothiophen-2-yl-ph enyl)amine: Compound (44)>

5.0 g of (4-benzothiazol-2-yl-phenyl)-(4-dibenzothiophen-2-yl-phenyl)-amine, 3.3 g of 2-(4-bromo-phenyl)benzothiophene, 0.2 g of tris(dibenzylideneacetone)dipalladium(0), 0.2 g of tri-t-butylphosphine, and 1.5 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using a toluene solvent to obtain 6.1 g (with a yield of 83.9%) of a yellow powder of (4-benzothiophen-2-yl-phenyl)-(4-benzothiazol-2-yl-phenyl)-(4-dibenzothiophen-2-yl-ph enyl)amine: Compound (44).

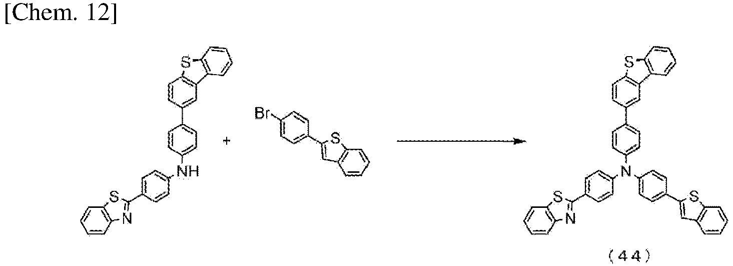

The structure of the obtained yellow powder was identified using NMR.

In ¹H-NMR (CDCl₃), the following signals of 28 hydrogens were detected.
δ (ppm) = 8.36 (1H), 8.23 (1H), 8.05 (1H), 8.00 (2H), 7.92 (1H), 7.89 (2H), 7.83 (1H), 7.77 (1H), 7.71 (1H), 7.68 (4H), 7.51 (1H), 7.48 (3H), 7.36 (2H), 7.32 (3H), 7.27 (4H).

### Example 10

### < Synthesis of (4-benzofuran-2-yl-phenyl)-(4-benzothiazol-2-yl-phenyl)-(4-dibenzofuran-3-yl-phenyl)a mine: Compound (55)>

5.0 g of (4-benzothiazol-2-yl-phenyl)-(4-dibenzofuran-3-yl-phenyl)-amine, 3.2 g of 2-(4-bromo-phenyl)benzofuran, 0.2 g of tris(dibenzylideneacetone)dipalladium(0), 0.2 g of tri-t-butylphosphine, and 1.5 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through crystallization using a toluene/acetone mixed solvent to obtain 6.2 g (with a yield of 87.9%) of a yellow powder of (4-benzofuran-2-yl-phenyl)-(4-benzothiazol-2-yl-phenyl)-(4-dibenzofuran-3-yl-phenyl)a mine: Compound (55).

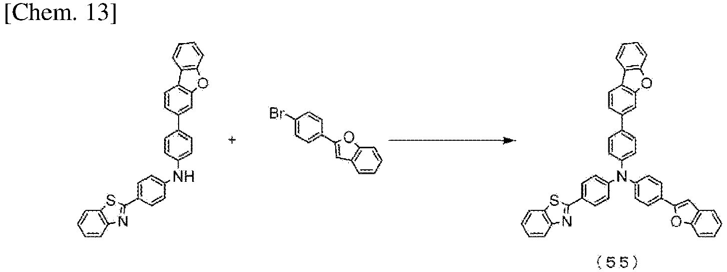

The structure of the obtained yellow powder was identified using NMR.

In ¹H-NMR (CDCl₃), the following signals of 28 hydrogens were detected.
δ (ppm) = 8.04 (1H), 8.01 (3H), 7.98 (1H), 7.89 (1H), 7.82 (2H), 7.80 (1H), 7.66 (2H), 7.60 (2H), 7.58 (1H), 7.52 (1H), 7.48 (2H), 7.37 (2H), 7.33-7.26 (6H), 7.24 (2H), 6.98 (1H).

### Example 11

### < Synthesis of (4-benzofuran-2-yl-phenyl)-(4-benzothiazol-2-yl-phenyl)-(4-dibenzothiophen-2-yl-phenyl )amine: Compound (59)>

5.0 g of (4-benzothiazol-2-yl-phenyl)-(4-dibenzothiophen-2-yl-phenyl)-amine, 3.1 g of 2-(4-bromo-phenyl)benzofuran, 0.2 g of tris(dibenzylideneacetone)dipalladium(0), 0.2 g of tri-t-butylphosphine, and 1.5 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through crystallization using a toluene/acetone mixed solvent to obtain 5.4 g (with a yield of 77.4%) of a yellow powder of (4-benzofuran-2-yl-phenyl)-(4-benzothiazol-2-yl-phenyl)-(4-dibenzothiophen-2-yl-phenyl )amine: Compound (59).

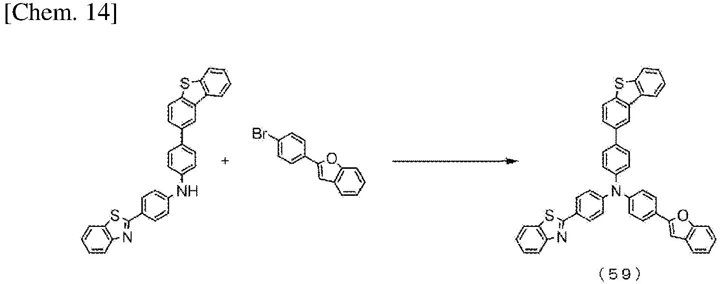

The structure of the obtained yellow powder was identified using NMR.

In ¹H-NMR (CDCl₃), the following signals of 28 hydrogens were detected.
δ (ppm) = 8.37 (1H), 8.24 (1H), 8.05 (1H), 8.01 (2H), 7.93 (1H), 7.89 (2H), 7.82 (2H), 7.72 (1H), 7.69 (2H), 7.58 (1H), 7.53 (1H), 7.49 (3H), 7.37 (1H), 7.32 (2H), 7.29 (2H), 7.27 (3H), 7.24 (1H), 6.98 (1H).

### Example 12

### < Synthesis of (4' -benzofuran-2-yl-biphenyl-4-yl)-(4-benzoxazol-2-yl-phenyl)-(4-naphthalen-2-yl-pheny l)amine: Compound (69)>

9.0 g of (4-benzoxazol-2-yl-phenyl)-(4-naphthalen-2-yl-phenyl)-amine, 7.3 g of 2-(4'-chloro-biphenyl-4-yl)benzofuran, 0.4 g of tris(dibenzylideneacetone)dipalladium(0), 0.2 g of tri-t-butylphosphine, and 4.2 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through crystallization using a THF/acetone mixed solvent to obtain 9.8 g (with a yield of 65.8%) of a yellow powder of (4' -benzofuran-2-yl-biphenyl-4-yl)-(4-benzoxazol-2-yl-phenyl)-(4-naphthalen-2-yl-pheny l)amine: Compound (69).

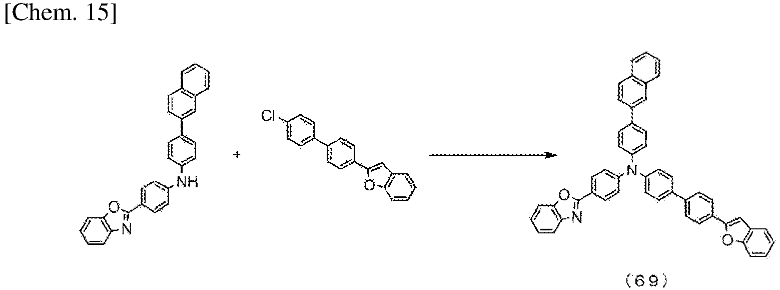

The structure of the obtained yellow powder was identified using NMR.

In ¹H-NMR (CDCl₃), the following signals of 32 hydrogens were detected.
δ (ppm) = 8.14 (2H), 8.06 (1H), 7.94 (3H), 7.91 (1H), 7.87 (1H), 7.76 (2H), 7.71 (4H), 7.64 (2H), 7.60 (1H), 7.58-7.46 (4H), 7.33 (5H), 7.29 (3H), 7.25 (2H), 7.07 (1H).

### Example 13

### < Synthesis of (4' -benzofuran-2-yl-biphenyl-4-yl)-(4-benzoxazol-2-yl-phenyl)-(4-phenanthren-9-yl-phen yl)amine: Compound (70)>

10.0 g of (4-benzoxazol-2-yl-phenyl)-(4-phenanthren-9-yl-phenyl)-amine, 7.2 g of 2-(4'-chloro-biphenyl-4-yl)benzofuran, 0.4 g of tris(dibenzylideneacetone)dipalladium(0), 0.2 g of tri-t-butylphosphine, and 3.1 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 13.2 g (with a yield of 83.5%) of a yellow powder of
(4' -benzofuran-2-yl-biphenyl-4-yl)-(4-benzoxazol-2-yl-phenyl)-(4-phenanthren-9-yl-phen yl)amine: Compound (70).

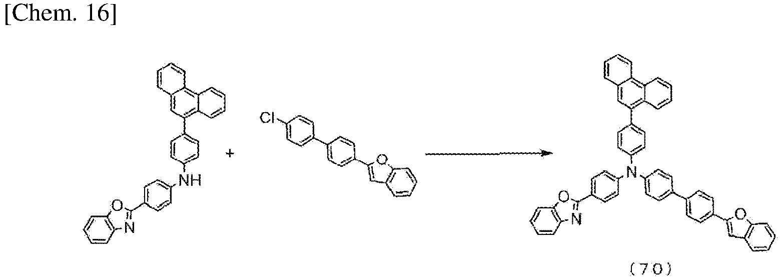

The structure of the obtained yellow powder was identified using NMR.

In ¹H-NMR (CDCl₃), the following signals of 34 hydrogens were detected.
δ (ppm) = 8.80 (1H), 8.74 (1H), 8.18 (2H), 8.07 (1H), 7.96 (2H), 7.92 (1H), 7.75 (5H), 7.67 (3H), 7.60 (4H), 7.55 (3H), 7.37 (4H), 7.32 (4H), 7.29 (1H), 7.25 (1H), 7.08 (1H).

### Example 14

### < Synthesis of (4' -benzofuran-2-yl-biphenyl-4-yl)-(4-benzoxazol-2-yl-phenyl)-(4-dibenzofuran-3-yl-phe nyl)amine: Compound (72)>

10.0 g of (4-benzoxazol-2-yl-phenyl)-(4-dibenzofuran-3-yl-phenyl)-amine, 7.4 g of 2-(4'-chloro-biphenyl-4-yl)benzofuran, 0.4 g of tris(dibenzylideneacetone)dipalladium(0), 0.2 g of tri-t-butylphosphine, and 4.3 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through crystallization using a THF/acetone mixed solvent to obtain 10.3 g (with a yield of 66.0%) of a yellow powder of
(4' -benzofuran-2-yl-biphenyl-4-yl)-(4-benzoxazol-2-yl-phenyl)-(4-dibenzofuran-3-yl-phe nyl)amine: Compound (72).

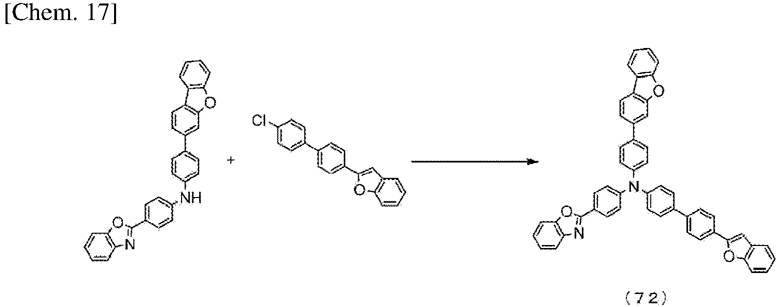

The structure of the obtained yellow powder was identified using NMR.

In ¹H-NMR (CDCl₃), the following signals of 32 hydrogens were detected.
δ (ppm) = 8.14 (2H), 8.00 (1H), 7.97 (1H), 7.95 (2H), 7.80 (1H), 7.75 (1H), 7.69 (4H), 7.62 (4H), 7.58 (3H), 7.47 (1H), 7.40-7.26 (10H), 7.24 (1H), 7.07 (1H).

### Example 15

### < Synthesis of (4' -benzofuran-2-yl-biphenyl-4-yl)-(4-benzoxazol-2-yl-phenyl)-(4-dibenzofuran-2-yl-phe nyl)amine: Compound (73)>

9.0 g of (4-benzoxazol-2-yl-phenyl)-(4-dibenzofuran-2-yl-phenyl)-amine, 6.7 g of 2-(4'-chloro-biphenyl-4-yl)benzofuran, 0.4 g of tris(dibenzylideneacetone)dipalladium(0), 0.2 g of tri-t-butylphosphine, and 2.9 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 12.0 g (with a yield of 83.6%) of a yellow powder of
(4' -benzofuran-2-yl-biphenyl-4-yl)-(4-benzoxazol-2-yl-phenyl)-(4-dibenzofuran-2-yl-phe nyl)amine: Compound (73).

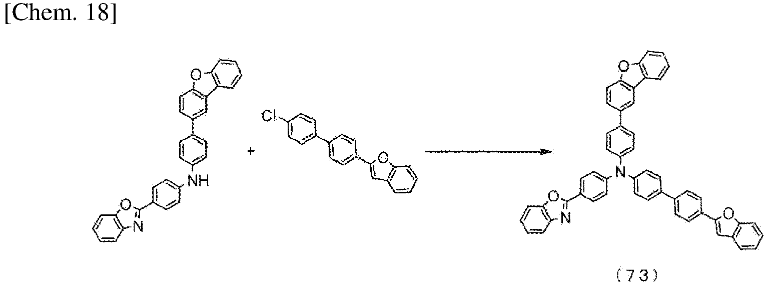

The structure of the obtained yellow powder was identified using NMR.

In ¹H-NMR (CDCl₃), the following signals of 32 hydrogens were detected.
δ (ppm) = 8.17 (1H), 8.15 (2H), 8.01 (1H), 7.95 (2H), 7.75 (1H), 7.70 (3H), 7.66 (2H), 7.63 (3H), 7.60 (2H), 7.56 (1H), 7.54 (1H), 7.49 (1H), 7.37 (1H), 7.35-7.26 (9H), 7.24 (1H), 7.07 (1H).

### Example 16

### < Synthesis of (4'-benzofuran-2-yl-biphenyl-4-yl)-(4-benzoxazol-2-yl-phenyl)-(4-dibenzothiophen-2-yl-phenyl)amine: Compound (74)>

10.0 g of (4-benzoxazol-2-yl-phenyl)-(4-dibenzothiophen-2-yl-phenyl)-amine, 7.2 g of 2-(4'-chloro-biphenyl-4-yl)benzofuran, 0.4 g of tris(dibenzylideneacetone)dipalladium(0), 0.2 g of tri-t-butylphosphine, and 3.1 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 14.6 g (with a yield of 92.8%) of a yellow powder of
(4'-benzofuran-2-yl-biphenyl-4-yl)-(4-benzoxazol-2-yl-phenyl)-(4-dibenzothiophen-2-yl-phenyl)amine: Compound (74).

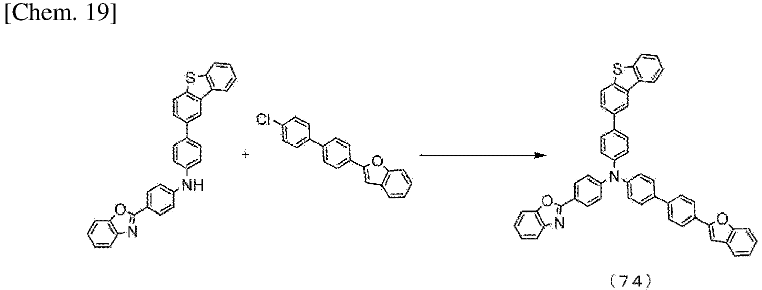

The structure of the obtained yellow powder was identified using NMR.

In ¹H-NMR (CDCl₃), the following signals of 32 hydrogens were detected.
δ (ppm) = 8.37 (1H), 8.23 (1H), 8.15 (2H), 7.95 (2H), 7.92 (1H), 7.88 (1H), 7.75 (1H), 7.71 (5H), 7.64 (2H), 7.60 (1H), 7.57 (1H), 7.54 (1H), 7.49 (2H), 7.37-7.26 (9H), 7.24 (1H), 7.07 (1H).

### Example 17

### < Synthesis of (4' -benzothiophen-2-yl-biphenyl-4-yl)-(4-benzoxazol-2-yl-phenyl)-(4-naphthalen-2-yl-ph enyl)amine: Compound (76)>

10.0 g of (4-benzoxazol-2-yl-phenyl)-(4-naphthalen-2-yl-phenyl)-amine, 8.6 g of 2-(4'-chloro-biphenyl-4-yl)benzothiophene, 0.5 g of tris(dibenzylideneacetone)dipalladium(0), 0.2 g of tri-t-butylphosphine, and 4.7 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through crystallization using a THF/acetone mixed solvent to obtain 11.1 g (with a yield of 65.7%) of a yellow powder of
(4'-benzothiophen-2-yl-biphenyl-4-yl)-(4-benzoxazol-2-yl-phenyl)-(4-naphthalen-2-yl-ph enyl)amine: Compound (76).

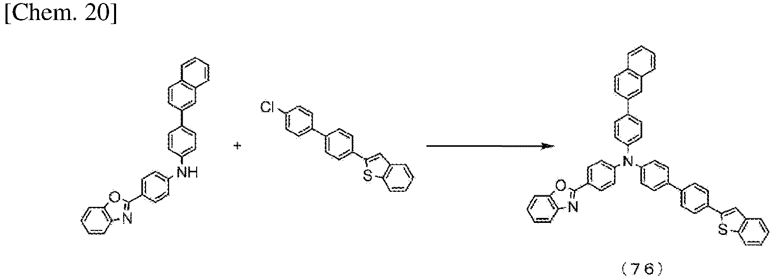

The structure of the obtained yellow powder was identified using NMR.

In ¹H-NMR (CDCl₃), the following signals of 32 hydrogens were detected.
δ (ppm) = 8.15 (2H), 8.06 (1H), 7.92 (2H), 7.86 (2H), 7.82-7.73 (5H), 7.71 (2H), 7.68 (2H), 7.63 (1H), 7.61 (2H), 7.57 (1H), 7.50 (2H), 7.40-7.26 (10H).

### Example 18

### < Synthesis of (4' -benzothiophen-2-yl-biphenyl-4-yl)-(4-benzoxazol-2-yl-phenyl)-(4-phenanthren-9-yl-p henyl)amine: Compound (78)>

10.0 g of (4-benzoxazol-2-yl-phenyl)-(4-phenanthren-9-yl-phenyl)-amine, 7.6 g of 2-(4'-chloro-biphenyl-4-yl)benzothiophene, 0.4 g of tris(dibenzylideneacetone)dipalladium(0), 0.2 g of tri-t-butylphosphine, and 3.1 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 13.1 g (with a yield of 81.1%) of a yellow powder of
(4'-benzothiophen-2-yl-biphenyl-4-yl)-(4-benzoxazol-2-yl-phenyl)-(4-phenanthren-9-yl-p henyl)amine: Compound (78).

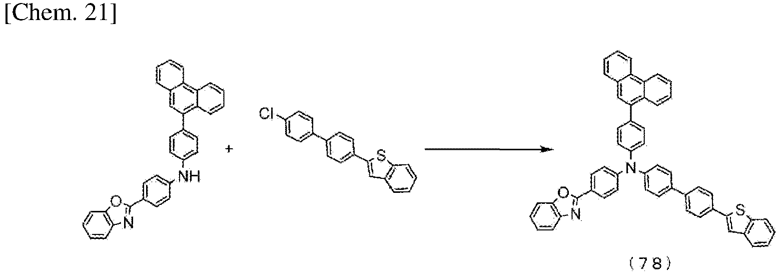

The structure of the obtained yellow powder was identified using NMR.

In ¹H-NMR (CDCl₃), the following signals of 34 hydrogens were detected.
δ (ppm) = 7.88-7.83 (4H), 7.79 (6H), 7.66 (4H), 7.59-7.55 (8H), 7.47 (1H), 7.38-7.30 (6H), 7.26-7.23 (5H).

### Example 19

### < Synthesis of (4'-benzothiophen-2-yl-biphenyl-4-yl)-(4-benzoxazol-2-yl-phenyl)-(4-dibenzofuran-2-yl-phenyl)amine: Compound (81)>

9.0 g of (4-benzoxazol-2-yl-phenyl)-(4-dibenzofuran-2-yl-phenyl)-amine, 7.0 g of 2-(4'-chloro-biphenyl-4-yl)benzothiophene, 0.4 g of tris(dibenzylideneacetone)dipalladium(0), 0.2 g of tri-t-butylphosphine, and 2.9 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 7.5 g (with a yield of 51.2%) of a yellow powder of
(4'-benzothiophen-2-yl-biphenyl-4-yl)-(4-benzoxazol-2-yl-phenyl)-(4-dibenzofuran-2-yl-phenyl)amine: Compound (81).

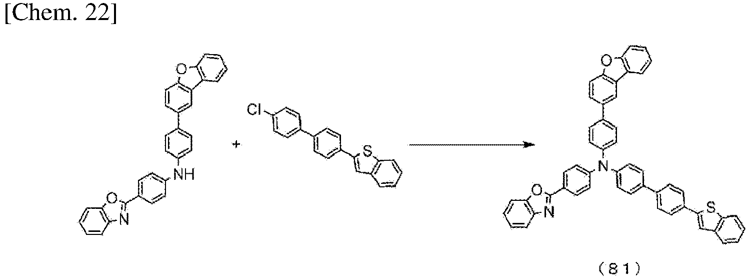

The structure of the obtained yellow powder was identified using NMR.

In ¹H-NMR (CDCl₃), the following signals of 32 hydrogens were detected.
δ (ppm) = 8.17 (1H), 8.15 (2H), 8.01 (1H), 7.84 (1H), 7.79 (3H), 7.75 (1H), 7.72-7.53 (11H), 7.49 (1H), 7.37 (2H), 7.36-7.29 (9H).

### Example 20

### < Synthesis of (4'-benzothiophen-2-yl-biphenyl-4-yl)-(4-benzoxazol-2-yl-phenyl)-(4-dibenzothiophen-2 -yl-phenyl)amine: Compound (82)>

10.0 g of (4-benzoxazol-2-yl-phenyl)-(4-dibenzothiophen-2-yl-phenyl)-amine, 7.5 g of 2-(4'-chloro-biphenyl-4-yl)benzothiophene, 0.4 g of tris(dibenzylideneacetone)dipalladium(0), 0.2 g of tri-t-butylphosphine, and 3.1 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 15.1 g (with a yield of 93.8%) of a yellow powder of
(4'-benzothiophen-2-yl-biphenyl-4-yl)-(4-benzoxazol-2-yl-phenyl)-(4-dibenzothiophen-2 -yl-phenyl)amine: Compound (82).

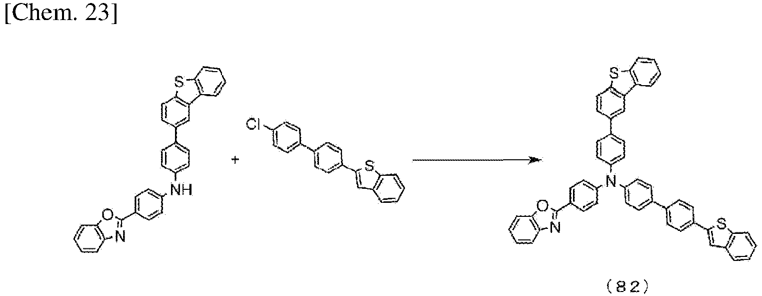

The structure of the obtained yellow powder was identified using NMR.

In ¹H-NMR (CDCl₃), the following signals of 32 hydrogens were detected.
δ (ppm) = 8.37 (1H), 8.23 (1H), 8.15 (2H), 7.92 (1H), 7.88 (1H), 7.84 (1H), 7.79 (3H), 7.75 (1H), 7.72 (1H), 7.69 (4H), 7.64 (1H), 7.61 (2H), 7.57 (1H), 7.49 (2H), 7.39-7.26 (10H).

### Example 21

### <Synthesis of (4-benzofuran-2-yl-phenyl)-(4'-benzoxazol-2-yl-biphenyl-4-yl)-(4-dibenzofuran-3-yl-phe nyl)amine: Compound (86)>

7.0 g of (4'-benzoxazol-2-yl-biphenyl-4-yl)-(4-dibenzofuran-3-yl-phenyl)amine, 5.3 g of 2-(4-bromophenyl)benzofuran, 0.5 g of tris(dibenzylideneacetone)dipalladium(0), 0.6 g of tri-t-butylphosphine, and 2.3 g of sodium t-butoxide were placed in a nitrogen-purged reaction vessel and stirred in a toluene solvent overnight under reflux. The reaction system was allowed to cool and then filtered, and the filtrate was concentrated to obtain a crude product. The obtained crude product was purified through crystallization using a toluene/acetone mixed solvent to obtain 7.2 g (with a yield of 64.8%) of a yellow powder of (4-benzofuran-2-yl-phenyl)-(4'-benzoxazol-2-yl-biphenyl-4-yl)-(4-dibenzofuran-3-yl-phe nyl)amine: Compound (86).

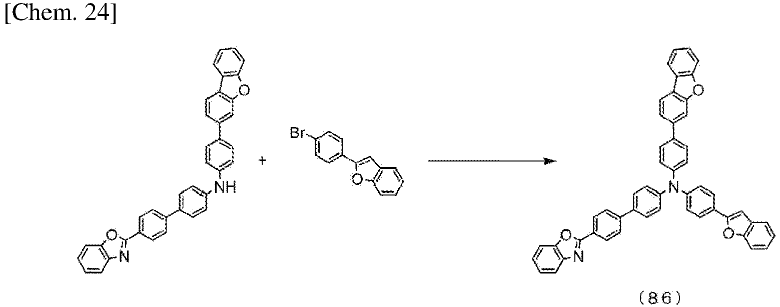

The structure of the obtained yellow powder was identified using NMR.

In ¹H-NMR (CDCl₃), the following signals of 32 hydrogens were detected.
δ (ppm) = 8.32 (2H), 7.99 (1H), 7.96 (1H), 7.80 (2H), 7.78 (2H), 7.77 (2H), 7.67-7.55 (8H), 7.51 (1H), 7.46 (1H), 7.38 (1H), 7.36 (2H), 7.29 (4H), 7.26 (3H), 7.22 (1H), 6.95 (1H).

### Example 22

### <Synthesis of (4-benzothiophen-2-yl-phenyl)-(4'-benzoxazol-2-yl-biphenyl-4-yl)-(4-dibenzofuran-3-yl-phenyl)amine: Compound (93)>

8.5 g of (4'-benzoxazol-2-yl-biphenyl-4-yl)-(4-dibenzofuran-3-yl-phenyl)amine, 5.3 g of 2-(4-bromo-phenyl)benzothiophene, 0.5 g of tris(dibenzylideneacetone)dipalladium(0), 0.6 g of tri-t-butylphosphine, and 2.3 g of sodium t-butoxide were placed in a nitrogen-purged reaction vessel and stirred in a xylene solvent overnight under reflux. The reaction system was allowed to cool and then filtered, and the filtrate was concentrated to obtain a crude product. The obtained crude product was purified through crystallization using a toluene/acetone mixed solvent to obtain 6.6 g (with a yield of 55.6%) of a yellow powder of (4-benzothiophen-2-yl-phenyl)-(4'-benzoxazol-2-yl-biphenyl-4-yl)-(4-dibenzofuran-3-yl-phenyl)amine: Compound (93).

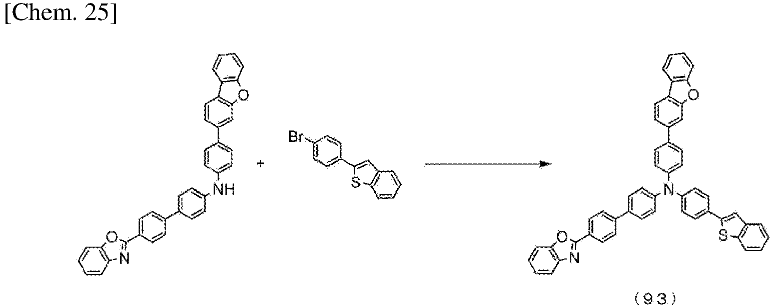

The structure of the obtained yellow powder was identified using NMR.

In ¹H-NMR (CDCl₃), the following signals of 32 hydrogens were detected.
δ (ppm) = 8.31 (2H), 7.98 (1H), 7.95 (1H), 7.81 (1H), 7.79 (2H), 7.75 (3H), 7.65 (2H), 7.63 (3H), 7.60 (3H), 7.57 (1H), 7.48 (1H), 7.46 (1H), 7.39-7.25 (9H), 7.23 (2H).

### Example 23

### <Synthesis of (7-benzothiophen-2-yl-naphthalen-2-yl)-(4-benzoxazol-2-yl-phenyl)-(4-dibenzofuran-3-yl -phenyl)amine: Compound (130)>

6.0 g of (4-benzoxazol-2-yl-phenyl)-(4-dibenzofuran-3-yl-phenyl)-amine, 5.0 g of 2-(2-bromo-naphthalen-7-yl)benzothiophene, 0.4 g of tris(dibenzylideneacetone)dipalladium(0), 0.3 g of tri-t-butylphosphine, and 1.9 g of sodium t-butoxide were placed in a nitrogen-purged reaction vessel and stirred in a toluene solvent for 7 hours under reflux. After the reaction system was allowed to cool, methanol was added thereto, and the precipitated solid was collected through filtration to obtain a crude product. The obtained crude product was purified through recrystallization using a monochlorobenzene solvent to obtain 8.5 g (with a yield of 90.1%) of a yellow powder of (7-benzothiophen-2-yl-naphthalen-2-yl)-(4-benzoxazol-2-yl-phenyl)-(4-dibenzofuran-3-yl -phenyl)amine: Compound (130).

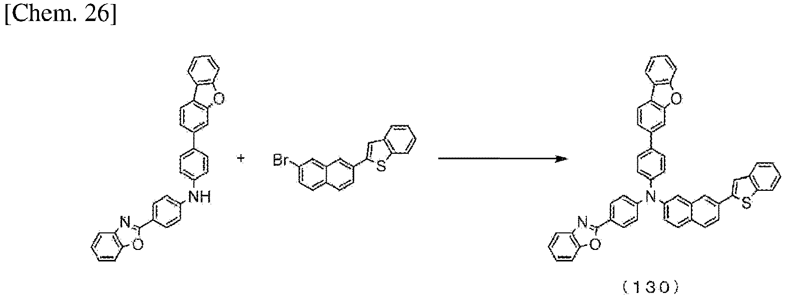

The structure of the obtained yellow powder was identified using NMR.

In ¹H-NMR (CDCl₃), the following signals of 30 hydrogens were detected.
δ (ppm) = 8.16 (2H), 8.01 (1H), 7.98 (1H), 7.97 (1H), 7.88-7.77 (6H), 7.75 (1H), 7.68 (2H), 7.66 (2H), 7.63 (1H), 7.58 (2H), 7.47 (1H), 7.42-7.31 (8H), 7.29 (2H).

### Example 24

### <Synthesis of (4-benzofuran-2-yl-phenyl)-(4-benzoxazol-2-yl-phenyl)-(4'-dibenzofuran-3-yl-biphenyl-4 -yl)amine: Compound (182)>

9.0 g of (4-benzoxazol-2-yl-phenyl)-(4-benzofuran-2-yl-phenyl)amine, 9.8 g of 3-(4'-bromo-biphenyl-4-yl)dibenzofuran, 0.6 g of tris(dibenzylideneacetone)dipalladium(0), 0.5 g of tri-t-butylphosphine, and 3.2 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 9.9 g (with a yield of 61.5%) of a yellow powder of (4-benzofuran-2-yl-phenyl)-(4-benzoxazol-2-yl-phenyl)-(4'-dibenzofuran-3-yl-biphenyl-4 -yl)amine: Compound (182).

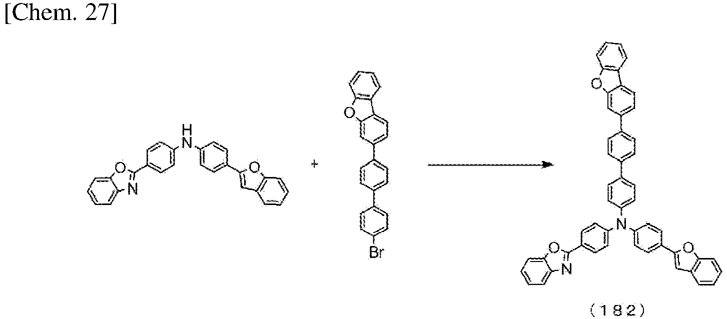

The structure of the obtained yellow powder was identified using NMR.

In ¹H-NMR (CDCl₃), the following signals of 32 hydrogens were detected.
δ (ppm) = 8.15 (2H), 8.01 (1H), 7.97 (1H), 7.84 (1H), 7.82 (2H), 7.78 (2H), 7.74 (1H), 7.72 (2H), 7.64 (3H), 7.58 (3H), 7.52 (1H), 7.47 (1H), 7.36 (1H), 7.33 (2H), 7.31-7.26 (7H), 7.23 (1H), 6.97 (1H).

### Example 25

### <Synthesis of (4-benzothiophen-2-yl-phenyl)-(4-benzoxazol-2-yl-phenyl)-(4'-dibenzofuran-3-yl-biphen yl-4-yl)amine: Compound (183)>

9.0 g of (4-benzoxazol-2-yl-phenyl)-(4-benzothiophen-2-yl-phenyl)amine, 9.4 g of 3-(4'-bromo-biphenyl-4-yl)dibenzofuran, 0.6 g of tris(dibenzylideneacetone)dipalladium(0), 0.4 g of tri-t-butylphosphine, and 3.1 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 15.0 g (with a yield of 95.0%) of a yellow powder of (4-benzothiophen-2-yl-phenyl)-(4-benzoxazol-2-yl-phenyl)-(4'-dibenzofuran-3-yl-biphen yl-4-yl)amine: Compound (183).

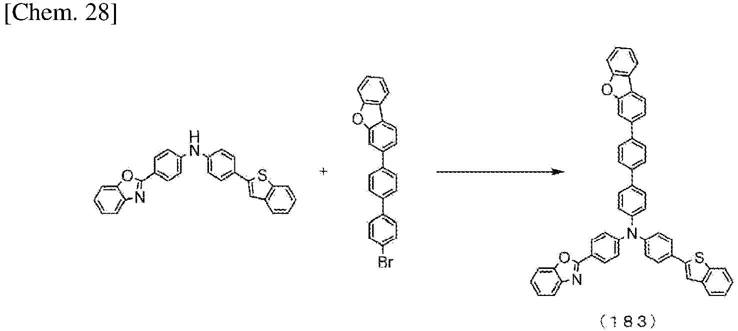

The structure of the obtained yellow powder was identified using NMR.

In ¹H-NMR (CDCl₃), the following signals of 32 hydrogens were detected.
δ (ppm) = 8.15 (2H), 8.01 (1H), 7.98 (1H), 7.84 (1H), 7.82 (1H), 7.80-7.70 (6H), 7.68 (2H), 7.64 (3H), 7.60 (1H), 7.56 (1H), 7.51 (1H), 7.47 (1H), 7.39-7.29 (6H), 7.29-7.23 (5H).

### Example 26

### <Synthesis of (4-benzothiophen-2-yl-phenyl)-(4-benzothiazol-2-yl-phenyl)-(4'-naphthalen-1-yl-bipheny l-4-yl)amine: Compound (186)>

5.0 g of (4-benzothiazol-2-yl-phenyl)-(4'-naphthalen-1-yl-biphenyl-4-yl)amine, 3.2 g of 2-(4-bromo-phenyl)benzothiophene, 0.2 g of tris(dibenzylideneacetone)dipalladium(0), 0.2 g of tri-t-butylphosphine, and 1.4 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through crystallization using a toluene/acetone mixed solvent to obtain 6.0 g (with a yield of 85.0%) of a yellow powder of (4-benzothiophen-2-yl-phenyl)-(4-benzothiazol-2-yl-phenyl)-(4'-naphthalen-1-yl-bipheny l-4-yl)amine: Compound (186).

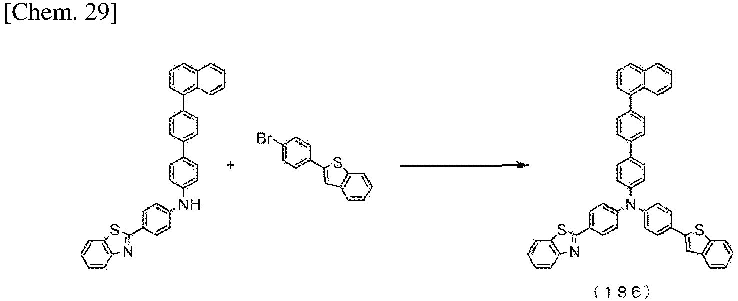

The structure of the obtained yellow powder was identified using NMR.

In ¹H-NMR (CDCl₃), the following signals of 32 hydrogens were detected. δ (ppm) = 8.05 (1H), 8.01 (2H), 7.99 (1H), 7.93 (1H), 7.89 (2H), 7.83 (1H), 7.77 (1H), 7.74 (2H), 7.68 (2H), 7.66 (2H), 7.60 (1H), 7.58 (1H), 7.54 (1H), 7.53-7.44 (5H), 7.36 (2H), 7.31 (3H), 7.26 (4H).

### Example 27

### <Synthesis of (4-benzofuran-2-yl-phenyl)-(4-benzothiazol-2-yl-phenyl)-(4'-naphthalen-1-yl-biphenyl-4-yl)amine: Compound (187)>

5.0 g of (4-benzothiazol-2-yl-phenyl)-(4'-naphthalen-1-yl-biphenyl-4-yl)amine, 3.0 g of 2-(4-bromo-phenyl)benzofuran, 0.2 g of tris(dibenzylideneacetone)dipalladium(0), 0.2 g of tri-t-butylphosphine, and 1.4 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through crystallization using a toluene/acetone mixed solvent to obtain 6.0 g (with a yield of 87.0%) of a yellow powder of (4-benzofuran-2-yl-phenyl)-(4-benzothiazol-2-yl-phenyl)-(4'-naphthalen-1-yl-biphenyl-4-yl)amine: Compound (187).

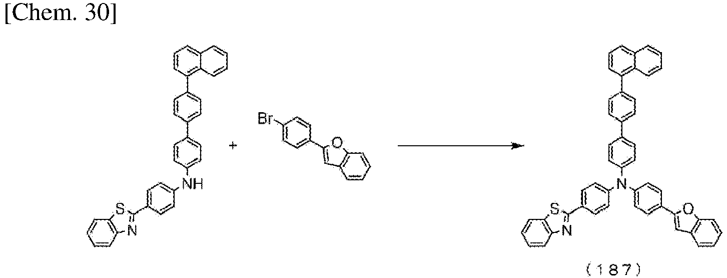

The structure of the obtained yellow powder was identified using NMR.

In ¹H-NMR (CDCl₃), the following signals of 32 hydrogens were detected. δ (ppm) = 8.05 (1H), 8.01 (2H), 7.99 (1H), 7.93 (1H), 7.89 (2H), 7.82 (2H), 7.74 (2H), 7.67 (2H), 7.60 (1H), 7.58 (2H), 7.53 (3H), 7.49 (1H), 7.47 (2H), 7.37 (1H), 7.30 (4H), 7.27 (3H), 7.24 (1H), 6.98 (1H).

### Example 28

### <Synthesis of (4-benzothiophen-2-yl-phenyl)-(4-benzoxazol-2-yl-phenyl)-{4-(1,10-phenanthrolin-2-yl)-phenyl}amine: Compound (188)>

7.5 g of (4-benzoxazol-2-yl-phenyl)-(4-benzothiophen-2-yl-phenyl)-amine, 5.7 g of 2-(4-chloro-phenyl)-1,10-phenanthroline, 0.2 g of tris(dibenzylideneacetone)dipalladium(0), 0.1 g of tri-t-butylphosphine, and 2.6 g of sodium t-butoxide were placed in a nitrogen-purged reaction vessel and stirred in a xylene solvent for 4 hours under reflux. After the reaction system was allowed to cool, methanol was added thereto, and the precipitated solid was collected through filtration to obtain a crude product. The obtained crude product was purified through crystallization using a toluene/acetone mixed solvent to obtain 2.8 g (with a yield of 23.0%) of a yellow powder of
(4-benzothiophen-2-yl-phenyl)-(4-benzoxazol-2)-yl-phenyl)-{4-(1,10-phenanthrolin-2-yl) -phenyl}amine: Compound (188).

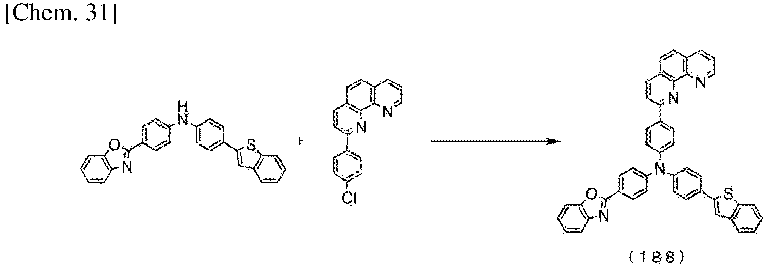

The structure of the obtained yellow powder was identified using NMR.

In ¹H-NMR (CDCl₃), the following signals of 28 hydrogens were detected. δ (ppm) = 9.23 (1H), 8.32 (1H), 8.30 (2H), 8.27 (1H), 8.17 (2H), 8.08 (1H), 7.83 (1H), 7.81 (1H), 7.77 (3H), 7.70 (2H), 7.64 (1H), 7.58 (1H), 7.54 (1H), 7.37 (2H), 7.34 (4H), 7.30 (2H), 7.27 (2H).

### Example 29

### <Synthesis of (4-benzothiophen-2-yl-phenyl)-(4-benzothiazol-2-yl-phenyl)-{4-(1,10-phenanthrolin-2-yl )-phenyl}amine: Compound (189)>

16.2 g of (4-benzothiazol-2-yl-phenyl)-(4-benzothiophen-2-yl-phenyl)-amine, 11.9 g of 2-(4-chloro-phenyl)-1,10-phenanthroline, 0.3 g of tris(dibenzylideneacetone)dipalladium(0), 0.3 g of tri-t-butylphosphine, and 5.4 g of sodium t-butoxide were placed in a nitrogen-purged reaction vessel and stirred in a xylene solvent for 4 hours under reflux. After the reaction system was allowed to cool, methanol was added thereto, and the precipitated solid was collected through filtration to obtain a crude product. The obtained crude product was purified through recrystallization using a monochlorobenzene solvent to obtain 13.0 g (with a yield of 51.0%) of a yellow powder of (4-benzothiophen-2-yl-phenyl)-(4-benzothiazol-2-yl-phenyl)-{4-(1,10-phenanthrolin-2-yl )-phenyl}amine: Compound (189).

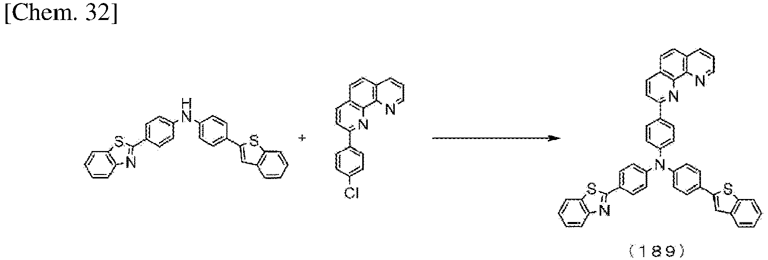

The structure of the obtained yellow powder was identified using NMR.

In ¹H-NMR (CDCl₃), the following signals of 28 hydrogens were detected. δ (ppm) = 9.23 (1H), 8.27 (1H), 8.30 (3H), 8.08 (1H), 8.05 (1H), 8.02 (2H), 7.90 (1H), 7.83 (2H), 7.78 (2H), 7.68 (2H), 7.64 (1H), 7.53 (1H), 7.49 (1H), 7.40-7.26 (9H).

### Example 30

### <Synthesis of (4-benzothiophen-2-yl-phenyl)-(4-benzothiazol-2-yl-phenyl)-(7-dibenzothiophen-2-yl-na phthalen-2-yl)amine: Compound (191)>

9.0 g of (4-benzothiazol-2-yl-phenyl)-(4-benzothiophen-2-yl-phenyl)amine, 8.9 g of 2-(2-bromo-naphthalen-7-yl)dibenzothiophene, 0.2 g of tris(dibenzylideneacetone)dipalladium(0), 0.1 g of tri-t-butylphosphine, and 3.1 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 15.0 g (with a yield of 95.0%) of a yellow powder of (4-benzothiophen-2-yl-phenyl)-(4-benzoxazol-2-yl-phenyl)-(4'-dibenzofuran-3-yl-biphen yl-4-yl)amine: Compound (191).

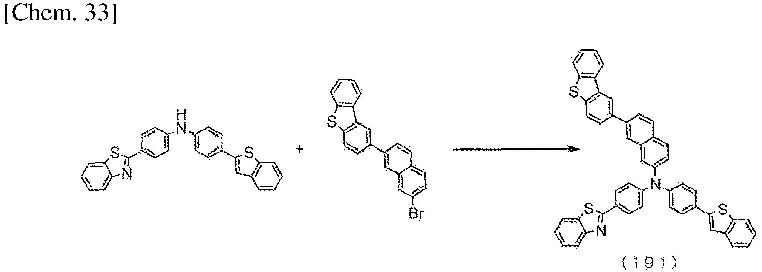

The structure of the obtained yellow powder was identified using NMR.

In ¹H-NMR (CDCl₃), the following signals of 30 hydrogens were detected. δ (ppm) = 8.44 (1H), 8.24 (1H), 8.04 (1H), 8.00 (2H), 7.99 (1H), 7.93 (2H), 7.90-7.77 (6H), 7.76 (1H), 7.68 (1H), 7.66 (2H), 7.50 (1H), 7.47 (3H), 7.40-7.29 (4H), 7.26 (4H).

### Example 31

### <Synthesis of (4'-benzothiophen-2-yl-biphenyl-4-yl)-(4-benzoxazol-2-yl-phenyl)-(4-dlibenzothiophen-4 -yl-phenyl)amine: Compound (192)>

10.0 g of (4-benzoxazol-2-yl-phenyl)-(4-dibenzothiophen-4-yl-phenyl)-amine, 7.5 g of 2-(4'-chloro-biphenyl-4-yl)benzothiophene, 0.4 g of tris(dibenzylideneacetone)dipalladium(0), 0.2 g of tri-t-butylphosphine, and 3.1 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 13.0 g (with a yield of 80.9%) of a yellow powder of
(4'-benzothiophen-2-yl-biphenyl-4-yl)-(4-benzoxazol-2-yl-phenyl)-(4-dibenzothiophen-4 -yl-phenyl)amine: Compound (192).

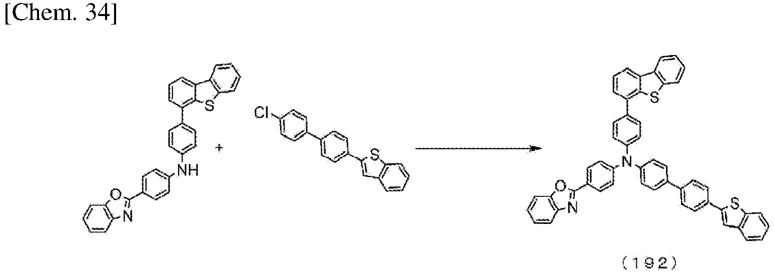

The structure of the obtained yellow powder was identified using NMR.

In ¹H-NMR (CDCl₃), the following signals of 32 hydrogens were detected. δ (ppm) = 8.22-8.16 (4H), 7.88-7.73 (8H), 7.69 (2H), 7.65 (2H), 7.61-7.48 (6H), 7.37-7.30 (10H).

### Example 32

### <Synthesis of (4'-benzofuran-2-yl-biphenyl-4-yl)-(4-benzoxazol-2-yl-phenyl)-(4-dibenzothiophen-4-yl-phenyl)amine: Compound (193)>

10.0 g of (4-benzoxazol-2-yl-phenyl)-(4-dibenzothiophen-4-yl-phenyl)-amine, 7.2 g of 2-(4'-chloro-biphenyl-4-yl)benzofuran, 0.4 g of tris(dibenzylideneacetone)dipalladium(0), 0.2 g of tri-t-butylphosphine, and 3.1 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 13.0 g (with a yield of 82.7%) of a yellow powder of
(4'-benzofuran-2-yl-biphenyl-4-yl)-(4-benzoxazol-2-yl-phenyl)-(4-dibenzothiophen-4-yl-phenyl)amine: Compound (193).

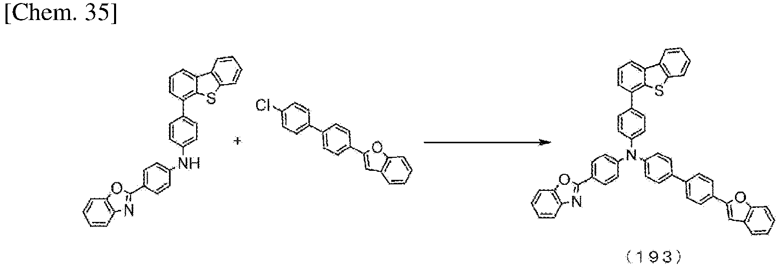

The structure of the obtained yellow powder was identified using NMR.

In ¹H-NMR (CDCl₃), the following signals of 32 hydrogens were detected. δ (ppm) = 8.21-8.15 (4H), 7.95 (2H), 7.86 (1H), 7.77-7.70 (5H), 7.65 (2H), 7.61-7.52 (5H), 7.48 (2H), 7.36-7.22 (10H), 7.07 (1H).

### Example 33

### <Synthesis of (4'-benzofuran-2-yl-biphenyl-4-yl)-(4-benzothiazol-2-yl-phenyl)-(4-naphthalen-1-yl-phen yl)amine: Compound (194)>

10.0 g of (4-benzothiazol-2-yl-phenyl)-(4-naphthalen-1-yl-phenyl)amine, 7.8 g of 2-(4'-chloro-biphenyl-4-yl)benzofuran, 0.6 g of tris(dibenzylideneacetone)dipalladium(0), 0.5 g of tri-t-butylphosphine, and 3.4 g of sodium t-butoxide were placed in a nitrogen-purged reaction vessel and stirred in a toluene solvent overnight under reflux. The reaction system was allowed to cool and then filtered, and the filtrate was concentrated to obtain a crude product. The obtained crude product was purified through recrystallization using a toluene solvent to obtain 4.0 g (with a yield of 24.6%) of a yellow powder of (4'-benzofuran-2-yl-biphenyl-4-yl)-(4-benzothiazol-2-yl-phenyl)-(4-naphthalen-1-yl-phen yl)amine: Compound (194).

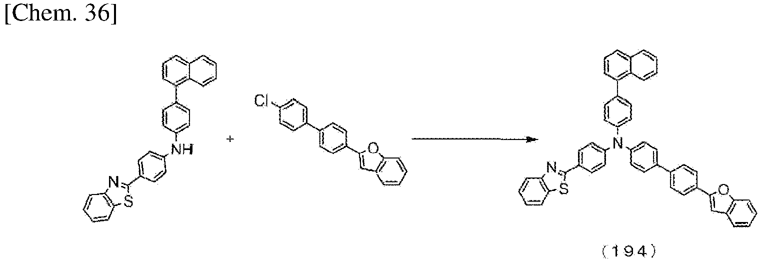

The structure of the obtained yellow powder was identified using NMR.

In ¹H-NMR (CDCl₃), the following signals of 32 hydrogens were detected.
δ (ppm) = 8.03 (2H), 8.01 (2H), 7.93 (3H), 7.88 (2H), 7.71 (2H), 7.64 (2H), 7.59 (1H), 7.57-7.44 (8H), 7.36 (1H), 7.35 (2H), 7.32 (2H), 7.29 (3H), 7.24 (1H), 7.06 (1H).

### Example 34

### <Synthesis of (4'-benzothiophen-2-yl-biphenyl-4-yl)-(4-benzothiazol-2-yl-phenyl)-(4-naphthalen-1-yl-p henyl)amine: Compound (195)>

10.0 g of (4-benzothiazol-2-yl-phenyl)-(4-naphthalen-1-yl-phenyl)amine, 8.2 g of 2-(4'-chloro-biphenyl-4-yl)benzothiophene, 0.6 g of tris(dibenzylideneacetone)dipalladium(0), 0.5 g of tri-t-butylphosphine, and 3.4 g of sodium t-butoxide were placed in a nitrogen-purged reaction vessel and stirred in a toluene solvent overnight under reflux. The reaction system was allowed to cool and then filtered, and the filtrate was concentrated to obtain a crude product. The obtained crude product was purified through recrystallization using a monochlorobenzene solvent to obtain 5.8 g (with a yield of 35.0%) of a yellow powder of (4'-benzothiophen-2-yl-biphenyl-4-yl)-(4-benzothiazol-2-yl-phenyl)-(4-naphthalen-1-yl-p henyl)amine: Compound (195).

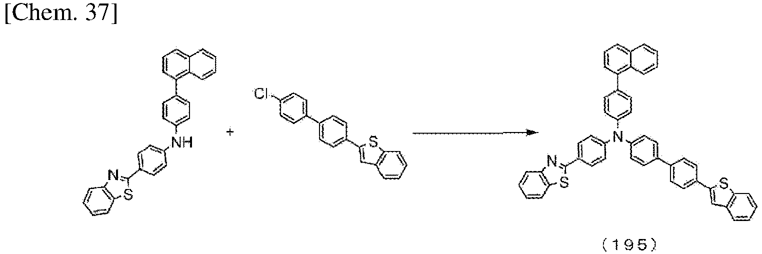

The structure of the obtained yellow powder was identified using NMR.

In ¹H-NMR (CDCl₃), the following signals of 32 hydrogens were detected.
δ (ppm) = 8.04 (2H), 8.01 (2H), 7.92 (1H), 7.88 (1H), 7.86 (1H), 7.84 (1H), 7.79 (3H), 7.67 (2H), 7.63 (2H), 7.59 (1H), 7.53 (1H), 7.51-7.44 (6H), 7.39-7.31 (7H), 7.29 (2H).

### Example 35

### <Synthesis of (4'-benzofuran-2-yl-biphenyl-4-yl)-(4-benzothiazol-2-yl-phenyl)-(4'-naphthalen-1-yl-bip henyl-4-yl)amine: Compound (196)>

2.4 g of (4-benzothiazol-2-yl-phenyl)-(4'-naphthalen-1-yl-biphenyl-4-yl)amine, 2.9 g of 2-(4'-chloro-biphenyl-4-yl)benzofuran, 0.1 g of tris(dibenzylideneacetone)dipalladium(0), 0.1 g of tri-t-butylphosphine, and 0.7 g of sodium t-butoxide were placed in a nitrogen-purged reaction vessel and stirred in a toluene solvent for 5 hours under reflux. After the reaction system was allowed to cool, methanol was added thereto, and the precipitated solid was collected through filtration to obtain a crude product. The obtained crude product was purified through crystallization using a monochlorobenzene/acetone mixed solvent to obtain 3.4 g (with a yield of 92.4%) of a yellow powder of (4'-benzofuran-2-yl-biphenyl-4-yl)-(4-benzothiazol-2-yl-phenyl)-(4'-naphthalen-1-yl-bip henyl-4-yl)amine: Compound (196).

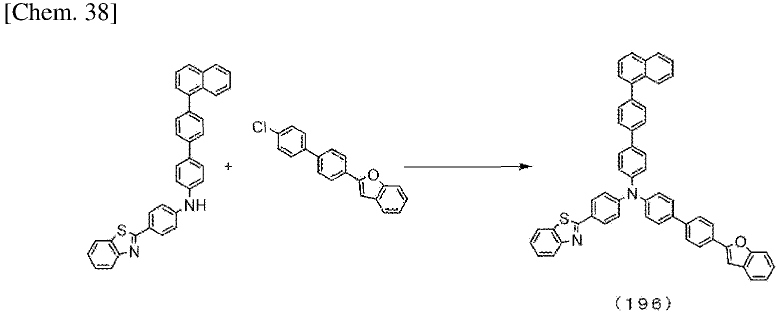

The structure of the obtained yellow powder was identified using NMR.

In ¹H-NMR (CDCl₃), the following signals of 36 hydrogens were detected.
δ (ppm) = 8.04 (1H), 8.00 (3H), 7.94 (2H), 7.92 (1H), 7.88 (2H), 7.74 (1H), 7.71 (2H), 7.68 (2H), 7.63 (2H), 7.62-7.56 (4H), 7.54 (2H), 7.47 (4H), 7.36 (1H), 7.33-7.21 (8H), 7.05 (1H).

### Example 36

### <Synthesis of (7-benzothiophen-2-yl-naphthalen-2-yl)-(4-benzothiazol-2-yl-phenyl)-(4-dibenzofuran-3-yl-phenyl)amine: Compound (197)>

10.0 g of (4-benzothiazol-2-yl-phenyl)-(4-dibenzofuran-3-yl-phenyl)-amine, 8.0 g of 2-(2-bromo-naphthalen-7-yl)benzothiophene, 0.6 g of tris(dibenzylideneacetone)dipalladium(0), 0.5 g of tri-t-butylphosphine, and 3.1 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 7.3 g (with a yield of 46.9%) of a yellow powder of (7-benzothiophen-2-yl-naphthalen-2-yl)-(4-benzothiazol-2-yl-phenyl)-(4-dibenzofuran-3-yl-phenyl)amine: Compound (197).

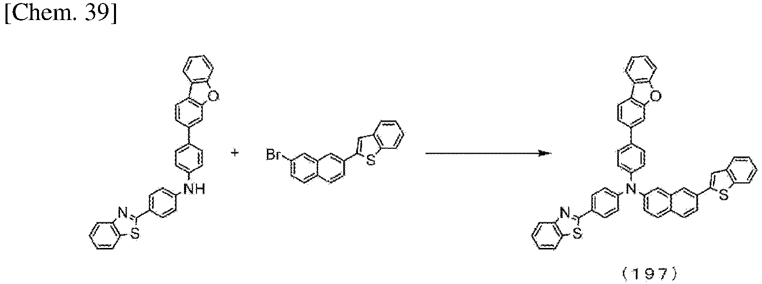

The structure of the obtained yellow powder was identified using NMR.

In ¹H-NMR (CDCl₃), the following signals of 30 hydrogens were detected. δ (ppm) = 8.05 (1H), 8.01 (1H), 8.00 (4H), 7.89 (1H), 7.85 (2H), 7.80 (4H), 7.67 (2H), 7.65 (2H), 7.63 (1H), 7.59 (1H), 7.48 (2H), 7.37 (4H), 7.33 (3H), 7.28 (2H).

### Example 37

### <Synthesis of (7-benzothiophen-2-yl-naphthalen-2-yl)-(4-benzothiazol-2-yl-phenyl)-(4-dibenzothiophen -2-yl-phenyl)amine: Compound (198)>

10.0 g of (4-benzothiazol-2-yl-phenyl)-(4-dibenzothiophen-2-yl-phenyl)-amine, 7.7 g of 2-(2-bromo-naphthalen-7-yl)benzothiophene, 0.2 g of tris(dibenzylideneacetone)dipalladium(0), 0.1 g of tri-t-butylphosphine, and 3.0 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 10.0 g (with a yield of 65.4%) of a yellow powder of (7-benzothiophen-2-yl-naphthalen-2-yl)-(4-benzothiazol-2-yl-phenyl)-(4-dibenzothiophen -2-yl-phenyl)amine: Compound (198).

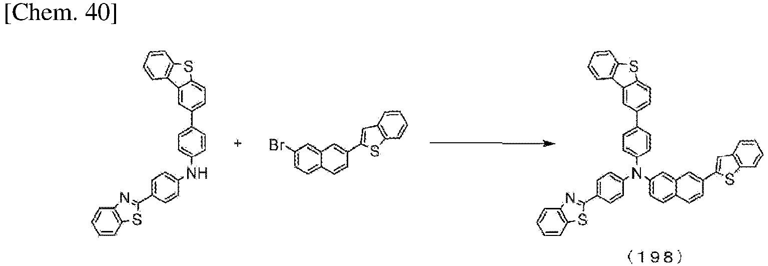

The structure of the obtained yellow powder was identified using NMR.

In ¹H-NMR (CDCl₃), the following signals of 30 hydrogens were detected. δ (ppm) = 8.37 (1H), 8.24 (1H), 8.05 (1H), 8.01 (2H), 7.98 (1H), 7.92 (1H), 7.90-7.76 (7H), 7.72 (1H), 7.69 (2H), 7.66 (1H), 7.65 (1H), 7.48 (3H), 7.40 (1H), 7.39-7.31 (5H), 7.28 (2H).

### Example 38

### <Synthesis of (7-benzothiophen-2-yl-naphthalen-2-yl)-{bis-(4-benzoxazol-2-yl-phenyl)}amine: Compound (201)>

7.0 g of bis(4-benzoxazol-2-yl-phenyl)amine, 6.5 g of 2-(2-bromo-naphthalen-7-yl)benzothiophene, 0.2 g of tris(dibenzylideneacetone)dipalladium(0), 0.1 g of tri-t-butylphosphine, and 2.5 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 6.8 g (with a yield of 59.1%) of a yellow powder of (7-benzothiophen-2-yl-naphthalen-2-yl)- {bis-(4-benzoxazol-2-yl-phenyl) } amine: Compound (201).

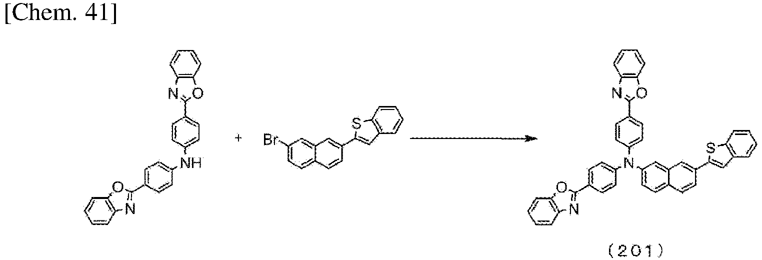

The structure of the obtained yellow powder was identified using NMR.

In ¹H-NMR (CDCl₃), the following signals of 27 hydrogens were detected. δ (ppm) = 8.20 (4H), 7.99 (1H), 7.87 (2H), 7.83 (2H), 7.79 (1H), 7.76 (2H), 7.68 (1H), 7.66 (1H), 7.58 (1H), 7.56 (1H), 7.39-7.28 (11H).

### Example 39

### <Synthesis of (7-benzothiophen-2-yl-naphthalen-2-yl)-(4-benzothiazol-2-yl-phenyl)-(4-benzothiophen-2-yl-phenyl)amine: Compound (202)>

7.0 g of (4-benzothiazol-2-yl-phenyl)-(4-benzothiophen-2-yl-phenyl)amine, 6.1 g of 2-(2-bromo-naphthalen-7-yl)benzothiophene, 0.4 g of tris(dibenzylideneacetone)dipalladium(0), 0.4 g of tri-t-butylphosphine, and 2.3 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 7.4 g (with a yield of 66.4%) of a yellow powder of (7-benzothiophen-2-yl-naphthalen-2-yl)-(4-benzothiazol-2-yl-phenyl)-(4-benzothiophen-2-yl-phenyl)amine: Compound (202).

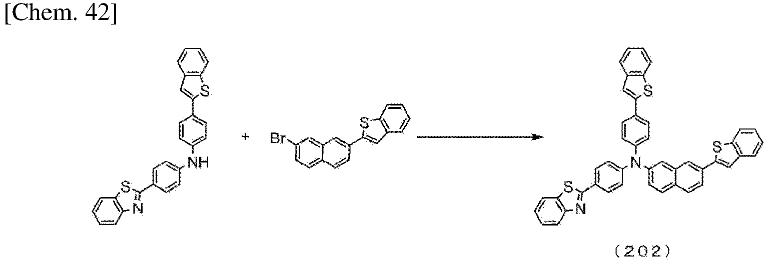

The structure of the obtained yellow powder was identified using NMR.

In ¹H-NMR (CDCl₃), the following signals of 28 hydrogens were detected. δ (ppm) = 8.04 (1H), 8.01 (2H), 7.97 (1H), 7.89 (1H), 7.87-7.75 (7H), 7.68 (2H), 7.66 (1H), 7.63 (1H), 7.52 (1H), 7.49 (1H), 7.40-7.29 (6H), 7.26 (4H).

### Example 40

### <Synthesis of (7-benzothiophen-2-yl-naphthalen-2-yl)-(4-benzothiazol-2-yl-phenyl)-(4-benzoxazol-2-yl -phenyl)amine: Compound (203)>

10.0 g of (4-benzothiazol-2-yl-phenyl)-(4-benzoxazol-2-yl-phenyl)amine, 8.9 g of 2-(2-bromo-naphthalen-7-yl)benzothiophene, 0.7 g of tris(dibenzylideneacetone)dipalladium(0), 0.6 g of tri-t-butylphosphine, and 3.5 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 9.9 g (with a yield of 61.3%) of a yellow powder of (7-benzothiophen-2-yl-naphthalen-2-yl)-(4-benzothiazol-2-yl-phenyl)-(4-benzoxazol-2-yl -phenyl)amine: Compound (203).

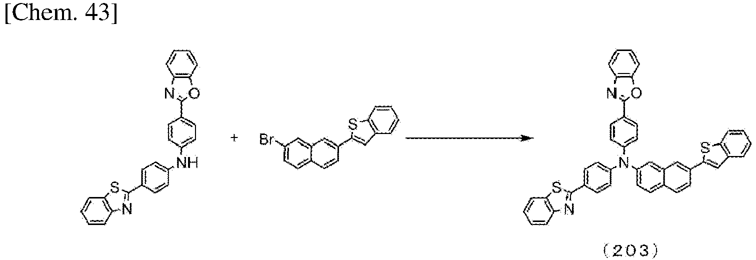

The structure of the obtained yellow powder was identified using NMR.

In ¹H-NMR (CDCl₃), the following signals of 27 hydrogens were detected. δ (ppm) = 8.18 (2H), 8.05 (1H), 8.04 (2H), 7.99 (1H), 7.93- 7.73 (7H), 7.67 (2H), 7.57 (1H), 7.49 (1H), 7.41-7.27 (10H).

### Example 41

### <Synthesis of bis(7-benzothiophen-2-yl-naphthalen-2-yl)-(4-benzoxazol-2-yl-phenyl)amine: Compound (204)>

4.5 g of 4-benzoxazol-2-yl-aniline, 15.2 g of 2-(2-bromo-naphthalen-7-yl)benzothiophene, 0.2 g of tris(dibenzylideneacetone)dipalladium(0), 0.1 g of tri-t-butylphosphine, and 5.1 g of sodium t-butoxide were placed in a nitrogen-purged reaction vessel and stirred in a xylene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 5.5 g (with a yield of 35.3%) of a yellow powder of bis {(7-benzothiophen-2-yl-naphthalen-2-yl)}-(4-benzoxazol-2-yl-phenyl)amine: Compound (204).

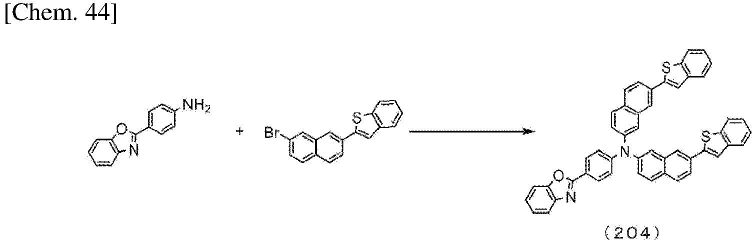

The structure of the obtained yellow powder was identified using NMR.

In ¹H-NMR (CDCl₃), the following signals of 30 hydrogens were detected.
δ (ppm) = 8.17 (2H), 7.97 (2H), 7.89-7.72 (11H), 7.65 (4H), 7.57 (1H), 7.40 (2H), 7.38-7.28 (8H).

### Example 42

### <Synthesis of {7-(4-benzoxazol-2-yl-phenyl)-naphthalen-2-yl}-(4-benzothiazol-2-yl-phenyl)-(4-benzoth iophen-2-yl-phenyl)amine: Compound (205)>

7.3 g of (4-benzothiazol-2-yl-phenyl)-(4-benzothiophen-2-yl-phenyl)amine, 7.4 g of 2-{4-(2-bromo-naphthalen-7-yl)-phenyl}benzoxazole, 0.5 g of tris(dibenzylideneacetone)dipalladium(0), 0.4 g of tri-t-butylphosphine, and 2.5 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 4.5 g (with a yield of 35.7%) of a yellow powder of {7-(4-benzoxazol-2-yl-phenyl)-naphthalen-2-yl}-(4-benzothiazol-2-yl-phenyl)-(4-benzoth iophen-2-yl-phenyl)amine: Compound (205).

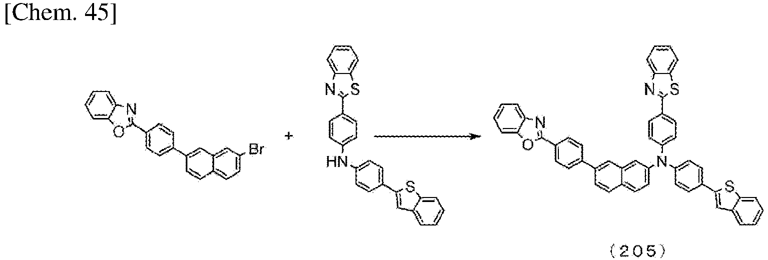

The structure of the obtained yellow powder was identified using NMR.

In ¹H-NMR (CDCl₃), the following signals of 31 hydrogens were detected.
δ (ppm) = 8.35 (2H), 8.05 (1H), 8.01 (2H), 7.96 (1H), 7.93 (1H), 7.90 (1H), 7.87 (2H), 7.84 (2H), 7.81 (1H), 7.77 (1H), 7.75 (1H), 7.68 (2H), 7.66 (1H), 7.60 (1H), 7.52 (1H), 7.49 (1H), 7.43-7.33 (5H), 7.32 (1H), 7.27 (4H).

### Example 43

### <Synthesis of {4-(7-benzothiophen-2-yl-naphthalen-2-yl)-phenyl}-(4-benzothiazol-2-yl-phenyl)-(4-ben zoxazol-2-yl-phenyl)amine: Compound (206)>

12.7 g of (4-benzothiazol-2-yl-phenyl)-(4-benzoxazol-2-yl-phenyl)amine, 12.4 g of 2-{2-(4-chloro-phenyl)-naphthalen-7-yl}benzothiophene, 0.3 g of tris(dibenzylideneacetone)dipalladium(0), 0.1 g of tri-t-butylphosphine, and 4.4 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using a monochlorobenzene solvent to obtain 7.8 g (with a yield of 34.2%) of a yellow powder of {4-(7-benzothiophen-2-yl-naphthalen-2-yl)-phenyl} -(4-benzothiazol-2-yl-phenyl)-(4-ben zoxazol-2-yl-phenyl)amine: Compound (206).

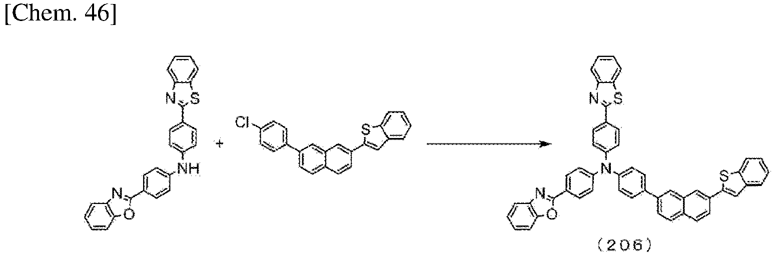

The structure of the obtained yellow powder was identified using NMR.

In ¹H-NMR (CDCl₃), the following signals of 31 hydrogens were detected. δ (ppm) = 8.22 (1H), 8.19 (2H), 8.12 (1H), 8.06 (1H), 8.05 (2H), 7.93 (2H), 7.91 (1H), 7.87 (2H), 7.82 (1H), 7.78 (1H), 7.75 (3H), 7.71 (1H), 7.58 (1H), 7.50 (1H), 7.38 (2H), 7.37-7.28 (9H).

### Example 44

### <Synthesis of {4-(7-benzothiophen-2-yl-naphthalen-2-yl)-phenyl}-(4-benzothiazol-2-yl-phenyl)-(4-ben zofuran-2-yl-phenyl)amine: Compound (207)>

8.5 g of (4-benzothiazol-2-yl-phenyl)-(4-benzofuran-2-yl-phenyl)amine, 8.3 g of 2-{2-(4-chloro-phenyl)-naphthalen-7-yl}benzothiophene, 0.2 g of tris(dibenzylideneacetone)dipalladium(0), 0.1 g of tri-t-butylphosphine, and 2.9 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using an o-dichlorobenzene solvent to obtain 7.8 g (with a yield of 51.0%) of a yellow powder of {4-(7-benzothiophen-2-yl-naphthalen-2-yl)-phenyl} -(4-benzothiazol-2-yl-phenyl)-(4-ben zofuran-2-yl-phenyl)amine: Compound (207).

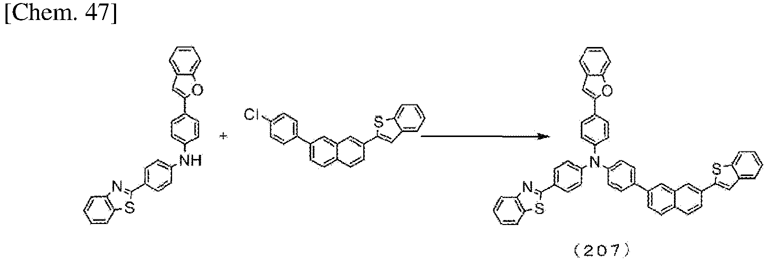

The structure of the obtained yellow powder was identified using NMR.

In ¹H-NMR (CDCl₃), the following signals of 32 hydrogens were detected. δ (ppm) = 8.21 (1H), 8.11 (1H), 8.05 (1H), 8.01 (2H), 7.90 (5H), 7.83 (3H), 7.77 (1H), 7.73 (2H), 7.71 (1H), 7.59 (1H), 7.53 (1H), 7.49 (1H), 7.41-7.27 (10H), 7.23 (1H), 6.99 (1H).

### Example 45

### <Synthesis of {4-(7-benzothiophen-2-yl-naphthalen-2-yl)-phenyl}-(4-benzothiazol-2-yl-phenyl)-(4-ben zothiophen-2-yl-phenyl)amine: Compound (208)>

7.3 g of (4-benzothiazol-2-yl-phenyl)-(4-benzothiophen-2-yl-phenyl)amine, 6.9 g of 2-{2-(4-chloro-phenyl)-naphthalen-7-yl}benzothiophene, 0.2 g of tris(dibenzylideneacetone)dipalladium(0), 0.1 g of tri-t-butylphosphine, and 2.4 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using an o-dichlorobenzene solvent to obtain 6.0 g (with a yield of 46.5%) of a yellow powder of {4-(7-benzothiophen-2-yl-naphthalen-2-yl)-phenyl}-(4-benzothiazol-2-yl-phenyl)-(4-ben zothiophen-2-yl-phenyl)amine: Compound (208).

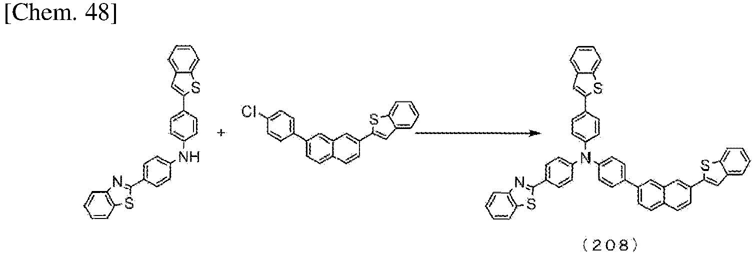

The structure of the obtained yellow powder was identified using NMR.

In ¹H-NMR (CDCl₃), the following signals of 32 hydrogens were detected. δ (ppm) = 8.22 (1H), 8.11 (1H), 8.06-8.00 (3H), 7.95-7.68 (15H), 7.53-7.47 (2H), 7.40-7.31 (7H), 7.28-7.26 (3H).

### Example 46

### <Synthesis of {4-(1-benzothiophen-2-yl-naphthalen-4-yl)-phenyl} -(4-benzothiazol-2-yl-phenyl)-(4-ben zoxazol-2-yl-phenyl)amine: Compound (209)>

7.3 g of (4-benzothiazol-2-yl-phenyl)-(4-benzoxazol-2-yl-phenyl)amine, 8.0 g of 2-{ 1-(4-bromo-phenyl)-naphthalen-4-yl}benzothiophene, 0.2 g of tris(dibenzylideneacetone)dipalladium(0), 0.1 g of tri-t-butylphosphine, and 2.5 g of sodium t-butoxide were placed in a nitrogen-purged reaction vessel and stirred in a toluene solvent overnight under reflux. After the reaction system was allowed to cool, methanol was added thereto, and the precipitated solid was collected through filtration to obtain a crude product. The obtained crude product was purified through recrystallization using an o-dichlorobenzene solvent to obtain 3.1 g (with a yield of 23.3%) of a yellow powder of {4-(1-benzothiophen-2-yl-naphthalen-4-yl)-phenyl} -(4-benzothiazol-2-yl-phenyl)-(4-ben zoxazol-2-yl-phenyl)amine: Compound (209).

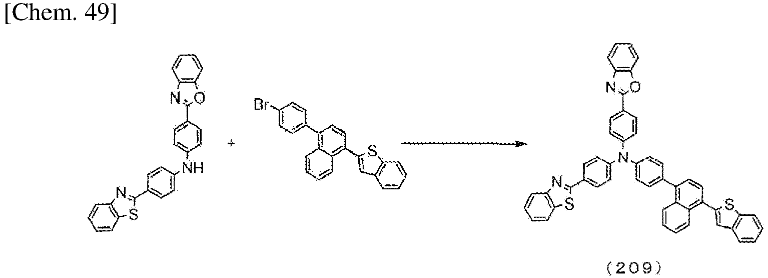

The structure of the obtained yellow powder was identified using NMR.

In ¹H-NMR (CDCl₃), the following signals of 31 hydrogens were detected. δ (ppm) = 8.39 (1H), 8.21 (2H), 8.10 (1H), 8.07 (2H), 8.06 (1H), 7.92 (1H), 7.90 (1H), 7.88 (1H), 7.77 (1H), 7.73 (1H), 7.61 (1H), 7.47 (7H), 7.47-7.32 (11H).

### Example 47

The melting point and the glass transition point of the compounds obtained in the examples were measured using a high-sensitivity differential scanning calorimeter (DSC3100SA manufactured by Bruker AXS K.K.). The measurement results are organized in Tables 1 and 2.

**Table 1**

| | Melting point | Glass transition point |
|---|---|---|
| Compound (54) | 246°C | 104°C |
| Compound (80) | 274°C | 132°C |
| Compound (8) | N.O. | 115°C |
| Compound (25) | 239°C | 118°C |
| Compound (28) | N.O. | 119°C |
| Compound (36) | 260°C | 122°C |
| Compound (40) | 252°C | 109°C |
| Compound (43) | N.O. | 118°C |
| Compound (44) | N.O. | 123°C |
| Compound (55) | N.O. | 115°C |
| Compound (59) | N.O. | 119°C |
| Compound (69) | 248°C | 119°C |
| Compound (70) | N.O. | 139°C |
| Compound (72) | 254°C | 128°C |
| Compound (73) | N.O. | 125°C |
| Compound (74) | N.O. | 131°C |
| Compound (76) | 271°C | 123°C |
| Compound (78) | N.O. | 142°C |
| Compound (81) | N.O. | 129°C |
| Compound (82) | N.O. | 134°C |
| Compound (86) | N.O. | 126°C |
| Compound (93) | N.O. | 130°C |
| Compound (130) | 261°C | 133°C |
| Compound (182) | 247°C | 126°C |
| Compound (183) | 269°C | 130°C |
| Compound (186) | N.O. | 124°C |

**Table 2**

| | Melting point | Glass transition point |
|---|---|---|
| Compound (187) | N.O. | 120°C |
| Compound (188) | N.O. | 146°C |
| Compound (189) | N.O. | 148°C |
| Compound (191) | N.O. | 140°C |
| Compound (192) | N.O. | 137°C |
| Compound (193) | N.O. | 133°C |
| Compound (194) | 243°C | 121°C |
| Compound (195) | 262°C | 125°C |
| Compound (196) | N.O. | 131°C |
| Compound (197) | N.O. | 132°C |
| Compound (198) | N.O. | 137°C |
| Compound (201) | 277°C | 134°C |
| Compound (202) | 271°C | 131°C |
| Compound (203) | 277°C | 133°C |
| Compound (204) | 300°C | 145°C |
| Compound (205) | 291°C | 144°C |
| Compound (206) | 310°C | 138°C |
| Compound (207) | 325°C | 135°C |
| Compound (208) | 337°C | 137°C |
| Compound (209) | N.O. | 135°C |

It is seen from the results that the compounds obtained in the examples had a glass transition point of 100°C or higher, which indicates that these compounds are stable in a thin film state.

### Example 48

A vapor-deposited film with a thickness of 80 nm was formed on a silicon substrate using each of the compounds obtained in the examples, and the refractive index n at wavelengths of 450 nm and 750 nm and the extinction coefficient k at wavelengths of 400 nm and 410 nm were measured using a spectroscopic measurement device (F10-RT-UV manufactured by Filmetrics). For comparison, the measurement was also performed on Comparative Compound (2-1) having the structural formula below and Alq₃ (see Patent Literature 4, for example). The measurement results are organized in Tables 3 to 5.

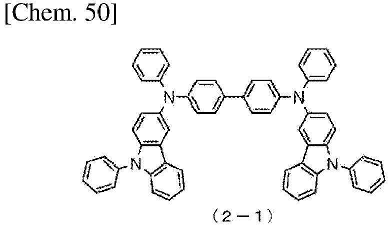

**Table 3**

| | Refractive index n (λ: 450 nm) | Refractive index n (λ: 750 nm) | Extinction coefficient k (λ: 400 nm) | Extinction coefficient k (λ: 410 nm) |
|---|---|---|---|---|
| Compound (54) | 2.48 | 1.91 | 1.08 | 0.89 |
| Compound (80) | 2.41 | 1.94 | 0.97 | 0.67 |
| Compound (8) | 2.38 | 1.91 | 1.00 | 0.69 |
| Compound (25) | 2.37 | 1.91 | 0.94 | 0.65 |
| Compound (28) | 2.40 | 1.92 | 1.02 | 0.72 |
| Compound (36) | 2.37 | 1.92 | 0.92 | 0.65 |
| Compound (40) | 2.52 | 1.92 | 1.13 | 0.96 |
| Compound (43) | 2.53 | 1.93 | 1.17 | 0.97 |
| Compound (44) | 2.54 | 1.94 | 1.07 | 0.93 |
| Compound (55) | 2.53 | 1.92 | 1.22 | 1.00 |
| Compound (59) | 2.52 | 1.92 | 1.07 | 0.90 |
| Compound (69) | 2.39 | 1.92 | 1.01 | 0.70 |
| Compound (70) | 2.34 | 1.91 | 0.86 | 0.60 |
| Compound (72) | 2.40 | 1.93 | 1.01 | 0.70 |
| Compound (73) | 2.35 | 1.92 | 0.94 | 0.64 |
| Compound (74) | 2.38 | 1.93 | 0.95 | 0.64 |
| Compound (76) | 2.39 | 1.93 | 0.94 | 0.66 |
| Compound (78) | 2.33 | 1.92 | 0.83 | 0.58 |

**Table 4**

| | Refractive index n (λ: 450 nm) | Refractive index n (λ: 750 nm) | Extinction coefficient k (λ: 400 nm) | Extinction coefficient k (λ: 410 nm) |
|---|---|---|---|---|
| Compound (81) | 2.37 | 1.93 | 0.94 | 0.64 |
| Compound (82) | 2.39 | 1.94 | 0.95 | 0.65 |
| Compound (86) | 2.43 | 1.93 | 1.07 | 0.79 |
| Compound (93) | 2.45 | 1.94 | 1.06 | 0.80 |
| Compound (130) | 2.30 | 1.93 | 0.61 | 0.41 |
| Compound (182) | 2.39 | 1.92 | 0.98 | 0.68 |
| Compound (183) | 2.39 | 1.93 | 0.97 | 0.69 |
| Compound (186) | 2.43 | 1.92 | 0.94 | 0.77 |
| Compound (187) | 2.47 | 1.90 | 1.15 | 0.89 |
| Compound (188) | 2.51 | 1.94 | 1.11 | 0.99 |
| Compound (189) | 2.59 | 1.97 | 1.08 | 1.06 |
| Compound (191) | 2.44 | 1.95 | 0.82 | 0.74 |
| Compound (192) | 2.36 | 1.93 | 0.85 | 0.58 |
| Compound (193) | 2.37 | 1.92 | 0.96 | 0.65 |
| Compound (194) | 2.44 | 1.92 | 0.96 | 0.81 |
| Compound (195) | 2.44 | 1.93 | 0.91 | 0.78 |
| Compound (196) | 2.46 | 1.92 | 1.00 | 0.82 |
| Compound (197) | 2.36 | 1.95 | 0.66 | 0.57 |
| Compound (198) | 2.39 | 1.96 | 0.67 | 0.59 |
| Compound (201) | 2.35 | 1.92 | 0.82 | 0.62 |
| Compound (202) | 2.41 | 1.96 | 0.74 | 0.66 |
| Compound (203) | 2.41 | 1.94 | 0.83 | 0.73 |
| Compound (204) | 2.27 | 1.94 | 0.49 | 0.33 |

**Table 5**

| | Refractive index n (λ: 450 nm) | Refractive index n (λ: 750 nm) | Extinction coefficient k (λ: 400 nm) | Extinction coefficient k (λ: 410 nm) |
|---|---|---|---|---|
| Compound (205) | 2.42 | 1.94 | 0.71 | 0.64 |
| Compound (206) | 2.44 | 1.96 | 0.87 | 0.74 |
| Compound (207) | 2.49 | 1.97 | 0.88 | 0.74 |
| Compound (208) | 2.48 | 1.98 | 0.82 | 0.71 |
| Compound (209) | 2.38 | 1.92 | 0.93 | 0.75 |
| Alq₃ | 1.88 | 1.73 | 0.16 | 0.14 |
| Compound (2-1) | 1.93 | 1.78 | 0.15 | 0.06 |

As shown in Tables 3 to 5, the refractive indices at wavelengths of 450 to 750 nm of Comparative Compound (2-1) and Alq₃ ranged from 1.73 to 1.93, whereas those of the compounds of the present invention ranged from 1.90 to 2.59. This indicates that an improvement in the light extraction efficiency of organic EL elements can be expected. Also, the extinction coefficients at wavelengths of 400 to 410 nm of Comparative Compound (2-1) and Alq₃ ranged from 0.06 to 0.16, whereas those of the compounds of the present invention ranged from 0.33 to 1.22. This indicates that the compounds of the present invention favorably absorb light rays with wavelengths of 400 to 410 nm of sunlight, thereby preventing them from affecting materials inside the elements.

### Example 49

With each of the compounds obtained in the examples, the absorbance was measured at wavelengths of 400 nm and 410 nm in a toluene solution adjusted to a concentration of 10⁻⁵ mol/L, an absorption spectrum was measured using a UV-NIR spectrophotometer (V-650 manufactured by JASCO Corporation) in toluene solutions adjusted to four different concentrations of 5×10⁻⁶ mol/L, 1×10⁻⁵ mol/L, 1.5×10⁻⁵ mol/L, and 2.0×10⁻⁵ mol/L, and the absorption coefficient was calculated from a calibration curve. For comparison, the measurement was also performed on Comparative Compound (2-1) above and Alq₃. The measurement results are organized in Tables 6 to 8.

**Table 6**

| | Peak wavelength (λmax) | Absorbance (λ: 400 nm) | Absorbance (λ: 410 nm) | Absorption coefficient |
|---|---|---|---|---|
| Compound (54) | 374 nm | 1.15 | 0.79 | 148418 |
| Compound (80) | 370 nm | 0.75 | 0.32 | 144532 |
| Compound (8) | 373 nm | 0.96 | 0.39 | 171764 |
| Compound (25) | 370 nm | 0.79 | 0.33 | 142715 |
| Compound (28) | 373 nm | 0.78 | 0.32 | 139073 |
| Compound (36) | 370 nm | 0.98 | 0.41 | 172886 |
| Compound (40) | 374 nm | 1.10 | 0.76 | 140210 |

**Table 7**

| | Peak wavelength (λmax) | Absorbance (λ: 400 nm) | Absorbance (λ: 410 nm) | Absorption coefficient |
|---|---|---|---|---|
| Compound (43) | 376 nm | 0.96 | 0.64 | 127899 |
| Compound (44) | 369 nm | 0.85 | 0.60 | 106893 |
| Compound (55) | 376 nm | 1.00 | 0.66 | 137401 |
| Compound (59) | 367 nm | 1.07 | 0.75 | 136598 |
| Compound (69) | 369 nm | 0.76 | 0.33 | 144071 |
| Compound (70) | 362 nm | 0.65 | 0.25 | 129057 |
| Compound (72) | 370 nm | 0.82 | 0.35 | 155525 |
| Compound (73) | 363 nm | 0.95 | 0.42 | 173650 |
| Compound (74) | 366 nm | 0.67 | 0.29 | 126460 |
| Compound (76) | 369 nm | 0.67 | 0.29 | 127809 |
| Compound (78) | 363 nm | 0.76 | 0.29 | 151523 |
| Compound (81) | 363 nm | 0.57 | 0.25 | 105312 |
| Compound (82) | 366 nm | 0.79 | 0.34 | 151205 |
| Compound (86) | 377 nm | 0.91 | 0.47 | 151279 |
| Compound (93) | 376 nm | 0.95 | 0.50 | 153247 |
| Compound (130) | 346 nm | 0.56 | 0.20 | 144262 |
| Compound (182) | 370 nm | 1.05 | 0.43 | 191603 |
| Compound (183) | 370 nm | 0.81 | 0.34 | 146263 |
| Compound (186) | 370 nm | 1.15 | 0.79 | 148993 |
| Compound (187) | 369 nm | 1.00 | 0.69 | 132179 |
| Compound (188) | 385 nm | 1.02 | 0.55 | 123739 |
| Compound (189) | 389 nm | 1.04 | 0.77 | 111667 |
| Compound (191) | 386 nm | 1.14 | 0.76 | 133177 |
| Compound (192) | 366 nm | 0.59 | 0.22 | 127787 |
| Compound (193) | 365 nm | 0.63 | 0.23 | 132819 |
| Compound (194) | 363 nm | 0.89 | 0.55 | 115452 |
| Compound (195) | 364 nm | 0.76 | 0.47 | 97035 |

**Table 8**

| | Peak wavelength (λmax) | Absorbance (λ: 400 nm) | Absorbance (λ: 410 nm) | Absorption coefficient |
|---|---|---|---|---|
| Compound (196) | 368 nm | 1.14 | 0.75 | 158260 |
| Compound (197) | 345 nm | 0.84 | 0.51 | 131806 |
| Compound (198) | 381 nm | 0.60 | 0.37 | 74166 |
| Compound (201) | 347 nm | 0.76 | 0.23 | 122430 |
| Compound (202) | 348 nm | 1.16 | 0.76 | 140200 |
| Compound (203) | 348 nm | 0.95 | 0.54 | 110282 |
| Compound (204) | 341 nm | 0.44 | 0.20 | 186228 |
| Compound (205) | 354 nm | 0.68 | 0.45 | 100940 |
| Compound (206) | 373 nm | 0.80 | 0.50 | 102688 |
| Compound (207) | 375 nm | 0.77 | 0.51 | 106589 |
| Compound (208) | 375 nm | 0.93 | 0.62 | 125282 |
| Compound (209) | 379 nm | 0.86 | 0.47 | 106660 |
| Alq₃ | 394 nm | 0.07 | 0.06 | 7518 |
| Compound (2-1) | 358 nm | 0.07 | 0.02 | 48856 |

As shown in Tables 6 to 8, the absorbance values at wavelengths of 400 to 410 nm of Comparative Compound (2-1) and Alq₃ ranged from 0.02 to 0.07, whereas the compounds of the present invention exhibited larger values ranging from 0.20 to 1.16. This indicates that the compounds of the present invention favorably absorb light rays with wavelengths of 400 to 410 nm of sunlight. With regard to the absorption coefficient as well, the compounds of the present invention had values of 70000 or more. This means that the compounds of the present invention absorb light better under the same concentration conditions. It is also indicated that the compounds of the present invention are materials that absorb light well even in a thin film state and provide excellent light resistance.

### Example 50

An organic EL element was prepared using the compound obtained in Example 1, and the properties of the organic EL element were evaluated.

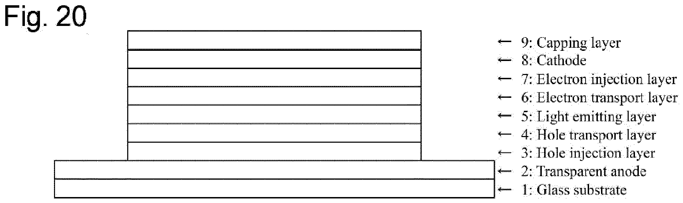

As shown in Fig. 20, the organic EL element was prepared by forming a reflecting ITO electrode serving as a metal anode 2 on a glass substrate 1 in advance, and furthermore, vapor-depositing a hole injection layer 3, a hole transport layer 4, a light emitting layer 5, an electron transport layer 6, an electron injection layer 7, a cathode 8, and a capping layer 9 in this order on the ITO electrode.

Specifically, a glass substrate 1 on which an ITO film with a thickness of 50 nm, a reflective silver alloy film with a thickness of 100 nm, and an ITO film with a thickness of 5 nm were formed in this order was ultrasonically cleaned in isopropyl alcohol for 20 minutes, and then dried for 10 minutes on a hot plate heated to 250°C. After that, UV/ozone treatment was performed for 2 minutes. Then, the glass substrate with ITO was attached inside a vacuum vapor deposition machine, and the pressure was reduced to 0.001 Pa or less.

Subsequently, an electron acceptor (Acceptor-1) having the structural formula below and Compound (3-1) having the structural formula below were vapor-deposited so as to cover the transparent anode 2 through binary vapor deposition at such vapor deposition rates that the ratio of the vapor deposition rate of Acceptor-1 to the vapor deposition rate of Compound (3-1) was 3:97, and a film with a thickness of 10 nm was thus formed as a hole injection layer 3.

A film of Compound (3-1) having the structural formula below was formed on the hole injection layer 3 as a hole transport layer 4 with a film thickness of 140 nm.

Compound (3-2) having the structural formula below and Compound (3-3) having the structural formula below were vapor-deposited on the hole transport layer 4 through binary vapor deposition at such vapor deposition rates that the ratio of the vapor deposition rate of (3-2) to the vapor deposition rate of (3-3) was 5:95, and a film with a thickness of 20 nm was thus formed as a light emitting layer 5.

Compound (3-4) having the structural formula below and Compound (3-5) having the structural formula below were vapor-deposited on the light emitting layer 5 through binary vapor deposition at such vapor deposition rates that the ratio of the vapor deposition rate of (3-4) to the vapor deposition rate of (3-5) was 50:50, and a film with a thickness of 30 nm was thus formed as an electron transport layer 6.

A film of lithium fluoride with a thickness of 1 nm was formed on this electron transport layer 6 as an electron injection layer 7.

A film of a magnesium-silver alloy with a thickness of 12 nm was formed on this electron injection layer 7 as a cathode 8.

Lastly, a film of Compound (54) of Example 1 with a thickness of 60 nm was formed as a capping layer 9.

The light emission properties of the prepared organic EL element when a DC voltage was applied thereto in the atmosphere at normal temperature were measured. Table 9 shows the results.

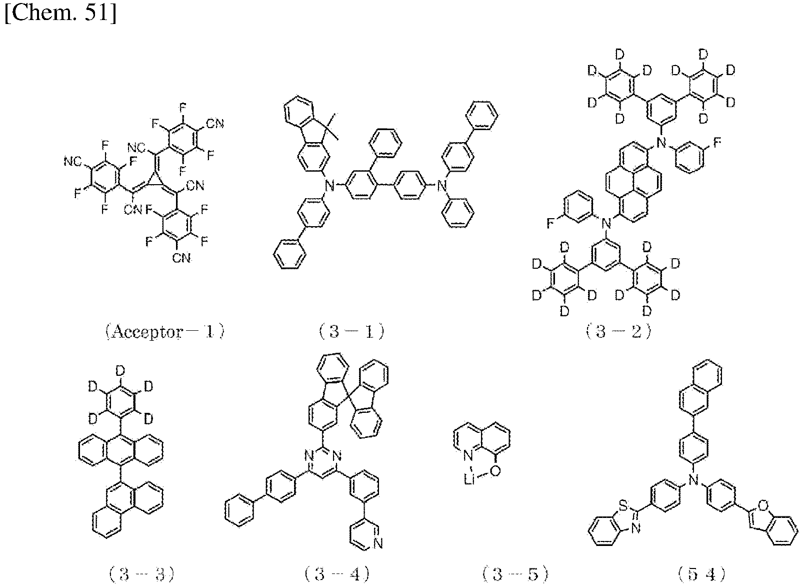

### Examples 51 to 95

Organic EL elements were prepared under similar conditions to those of Example 50, except that films of the respective compounds obtained in Examples 2 to 46 with a thickness of 60 nm were formed instead of the film of Compound (54) of Example 1 as the capping layer 9. The light emission properties of the prepared organic EL elements when a DC voltage was applied thereto in the atmosphere at normal temperature were measured. The results are organized in Tables 9 to 11.

### Comparative Example 1

For comparison, an organic EL element was prepared under similar conditions to those of Example 50, except that a film of Alq₃ with a thickness of 60 nm was formed instead of the film of Compound (54) of Example 1 as the capping layer 9. The light emission properties of the prepared organic EL element when a DC voltage was applied thereto in the atmosphere at normal temperature were measured. The results are organized in Table 11.

### Comparative Example 2

For comparison, an organic EL element was prepared under similar conditions to those of Example 50, except that a film of Comparative Compound (2-1) with a thickness of 60 nm was formed instead of the film of Compound (54) of Example 1 as the capping layer 9. The light emission properties of the prepared organic EL element when a DC voltage was applied thereto in the atmosphere at normal temperature were measured. The results are organized in Table 11.

The element lifespans of the organic EL elements prepared in the examples and comparative examples above were measured. The results are organized in Table 9 to 11. The element lifespan was defined as follows: when constant current driving was performed at 10 mA/cm² with the initial luminance being set to 100%, the time taken for the luminance to decay to 95% was measured as the element lifespan.

**Table 9**

| | Capping layer | Voltage [V] (@10 mA/cm²) | Luminance [cd/m²] (@10 mA/cm²) | Light emission efficiency [cd/A] (@10 mA/cm²) | Power efficiency [lm/W] (@10 mA/cm²) | Element lifespan decay to 95% |
|---|---|---|---|---|---|---|
| Ex. 50 | Compound (54) | 3.67 | 855 | 8.55 | 7.34 | 164 h |
| Ex. 51 | Compound (80) | 3.66 | 846 | 8.45 | 7.26 | 159 h |
| Ex. 52 | Compound (8) | 3.67 | 839 | 8.39 | 7.18 | 178 h |
| Ex. 53 | Compound (25) | 3.67 | 841 | 8.41 | 7.21 | 173 h |
| Ex. 54 | Compound (28) | 3.64 | 850 | 8.50 | 7.35 | 149 h |
| Ex. 55 | Compound (36) | 3.66 | 836 | 8.36 | 7.17 | 159 h |
| Ex. 56 | Compound (40) | 3.65 | 855 | 8.55 | 7.36 | 143 h |
| Ex. 57 | Compound (43) | 3.64 | 855 | 8.55 | 7.38 | 155 h |
| Ex. 58 | Compound (44) | 3.65 | 854 | 8.54 | 7.35 | 157 h |
| Ex. 59 | Compound (55) | 3.67 | 851 | 8.51 | 7.30 | 154 h |
| Ex. 60 | Compound (59) | 3.68 | 854 | 8.54 | 7.29 | 149 h |

**Table 10**

| | Capping layer | Voltage [V] (@10 mA/cm²) | Luminance [cd/m²] (@10 mA/cm²) | Light emission efficiency [cd/A] (@10 mA/cm²) | Power efficiency [lm/W] (@10 mA/cm²) | Element lifespan decay to 95% |
|---|---|---|---|---|---|---|
| Ex. 61 | Compound (69) | 3.64 | 838 | 8.38 | 7.23 | 141 h |
| Ex. 62 | Compound (70) | 3.67 | 835 | 8.35 | 7.15 | 173 h |
| Ex. 63 | Compound (72) | 3.63 | 841 | 8.41 | 7.28 | 154 h |
| Ex. 64 | Compound (73) | 3.65 | 843 | 8.43 | 7.26 | 153 h |
| Ex. 65 | Compound (74) | 3.67 | 836 | 8.36 | 7.16 | 144 h |
| Ex. 66 | Compound (76) | 3.66 | 840 | 8.40 | 7.22 | 152 h |
| Ex. 67 | Compound (78) | 3.67 | 833 | 8.33 | 7.14 | 176 h |
| Ex. 68 | Compound (81) | 3.64 | 835 | 8.35 | 7.20 | 176 h |
| Ex. 69 | Compound (82) | 3.68 | 838 | 8.38 | 7.16 | 170 h |
| Ex. 70 | Compound (86) | 3.62 | 848 | 8.48 | 7.37 | 143 h |
| Ex. 71 | Compound (93) | 3.62 | 846 | 8.46 | 7.35 | 176 h |
| Ex. 72 | Compound (130) | 3.63 | 831 | 8.31 | 7.18 | 158 h |
| Ex. 73 | Compound (182) | 3.63 | 839 | 8.39 | 7.27 | 173 h |
| Ex. 74 | Compound (183) | 3.63 | 836 | 8.37 | 7.25 | 167 h |
| Ex. 75 | Compound (186) | 3.63 | 842 | 8.42 | 7.29 | 168 h |
| Ex. 76 | Compound (187) | 3.65 | 847 | 8.47 | 7.30 | 167 h |
| Ex. 77 | Compound (188) | 3.64 | 850 | 8.50 | 7.33 | 142 h |
| Ex. 78 | Compound (189) | 3.68 | 862 | 8.62 | 7.35 | 150 h |
| Ex. 79 | Compound (191) | 3.61 | 848 | 8.48 | 7.38 | 176 h |
| Ex. 80 | Compound (192) | 3.64 | 832 | 8.32 | 7.19 | 144 h |
| Ex. 81 | Compound (193) | 3.63 | 836 | 8.36 | 7.24 | 145 h |
| Ex. 82 | Compound (194) | 3.62 | 846 | 8.46 | 7.34 | 166 h |
| Ex. 83 | Compound (195) | 3.65 | 844 | 8.44 | 7.27 | 141 h |
| Ex. 84 | Compound (196) | 3.62 | 849 | 8.48 | 7.36 | 174 h |
| Ex. 85 | Compound (197) | 3.64 | 837 | 8.38 | 7.23 | 175 h |
| Ex. 86 | Compound (198) | 3.67 | 836 | 8.36 | 7.16 | 154 h |
| Ex. 87 | Compound (201) | 3.64 | 846 | 8.46 | 7.31 | 169 h |
| Ex. 88 | Compound (202) | 3.67 | 846 | 8.45 | 7.24 | 158 h |
| Ex. 89 | Compound (203) | 3.62 | 840 | 8.40 | 7.30 | 162 h |
| Ex. 90 | Compound (204) | 3.64 | 831 | 8.31 | 7.16 | 161 h |
| Ex. 91 | Compound (205) | 3.66 | 843 | 8.43 | 7.23 | 141 h |
| Ex. 92 | Compound (206) | 3.63 | 844 | 8.44 | 7.30 | 170 h |

**Table 11**

| | Capping layer | Voltage [V] (@10 mA/cm²) | Luminance [cd/m²] (@10 mA/cm²) | Light emission efficiency [cd/A] (@10 mA/cm²) | Power efficiency [lm/W] (@10 mA/cm²) | Element lifespan decay to 95% |
|---|---|---|---|---|---|---|
| Ex. 93 | Compound (207) | 3.61 | 856 | 8.55 | 7.45 | 176 h |
| Ex. 94 | Compound (208) | 3.65 | 852 | 8.52 | 7.32 | 143 h |
| Ex. 95 | Compound (209) | 3.63 | 842 | 8.42 | 7.29 | 172 h |
| Com. Ex. 1 | Alq₃ | 3.65 | 714 | 7.14 | 6.15 | 114 h |
| Com. Ex. 2 | Compound (2-1) | 3.66 | 735 | 7.34 | 6.30 | 133 h |

As shown in Table 4, while the elements of Comparative Examples 1 and 2 and those of Examples 50 to 95 had substantially similar driving voltages at a current density of 10 mA/cm², the elements of Examples 50 to 95 exhibited improvements in the luminance, the light emission efficiency, the power efficiency, and the lifespan compared with the elements of Comparative Examples 1 and 2. This indicates that the light extraction efficiency can be significantly improved by including a material with a high refractive index that is suitably used in the organic EL element of the present invention in the capping layer.

### Industrial Applicability

An amine compound represented by the general formula (a) of the present invention has a high absorption coefficient and a high refractive index, can significantly improve the light extraction efficiency, and is stable in a thin film state, and thus, it is excellent as a compound for use in an organic EL element. An organic EL element in which a capping layer is formed using the compound can achieve good efficiency and can have improved durability and light resistance, with light rays of sunlight being absorbed and thereby prevented from affecting materials inside the element, and therefore can be used in applications such as home electric appliances and lighting equipment.

### List of Reference Numerals

- 1: Glass substrate
- 2: Transparent anode
- 3: Hole injection layer
- 4: Hole transport layer
- 5: Light emitting layer
- 6: Electron transport layer
- 7: Electron injection layer
- 8: Cathode
- 9: Capping layer

## Claims

1. An amine compound represented by a general formula (a) below: 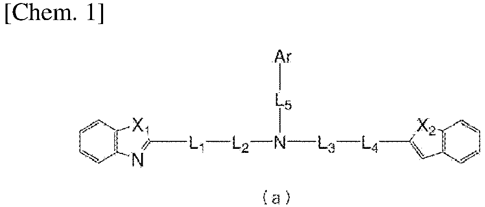
where Ar represents a substituted or unsubstituted aromatic hydrocarbon group, a substituted or unsubstituted aromatic heterocyclic group, or a substituted or unsubstituted fused polycyclic aromatic group;
L₁ to L₅ may be the same or different, and each represent a single bond, an unsubstituted divalent aromatic hydrocarbon group, an unsubstituted divalent aromatic heterocyclic group, or an unsubstituted divalent fused polycyclic aromatic group; and
X₁ and X₂ may be the same or different, and each represent an oxygen atom or a sulfur atom.

2. The amine compound according to claim 1, wherein L₁ in the general formula (a) represents an unsubstituted 1,4-phenylene group.

3. The amine compound according to claim 1, wherein L₂ to L₅ in the general formula (a) each represent a single bond, an unsubstituted phenylene group, an unsubstituted biphenylene group, or an unsubstituted naphthylene group.

4. The amine compound according to claim 3, wherein L₂ to L₅ in the general formula (a) each represent a single bond, an unsubstituted 1,4-phenylene group, an unsubstituted 4,4'-biphenylene group, an unsubstituted 2,6-naphthylene group, or an unsubstituted 2,7-naphthylene group.

5. The amine compound according to claim 1, wherein Ar in the general formula (a) represents a substituted or unsubstituted phenyl group, a substituted or unsubstituted biphenyl group, a substituted or unsubstituted naphthyl group, a substituted or unsubstituted phenanthrenyl group, a substituted or unsubstituted pyridyl group, a substituted or unsubstituted quinolyl group, a substituted or unsubstituted benzofuranyl group, a substituted or unsubstituted benzothienyl group, a substituted or unsubstituted dibenzofuranyl group, or a substituted or unsubstituted dibenzothienyl group.

6. The amine compound according to claim 5, wherein Ar in the general formula (a) represents an unsubstituted phenyl group, an unsubstituted 4-biphenyl group, an unsubstituted 2-naphthyl group, an unsubstituted 2-phenanthrenyl group, an unsubstituted 3-phenanthrenyl group, an unsubstituted 9-phenanthrenyl group, an unsubstituted 3-pyridyl group, an unsubstituted 3-quinolyl group, an unsubstituted 2-benzofuranyl group, an unsubstituted 2-benzothienyl group, an unsubstituted 2-dibenzofuranyl group, an unsubstituted 3-dibenzofuranyl group, an unsubstituted 2-dibenzothienyl group, or an unsubstituted 3-dibenzothienyl group.

7. An organic electroluminescent element comprising at least an anode electrode, a hole transport layer, a light emitting layer, an electron transport layer, a cathode electrode, and a capping layer arranged in this order, wherein the capping layer contains the amine compound according to any one of claims 1 to 6.

8. The organic electroluminescent element according to claim 7, wherein the capping layer has an extinction coefficient of 0.2 or more in a wavelength range of 400 to 410 nm, and the amine compound has an absorbance of 0.2 or more in a wavelength range of 400 to 410 nm in an absorption spectrum at a concentration of 10⁻⁵mol/L.

9. The organic electroluminescent element according to claim 7, wherein the capping layer has a refractive index of 1.85 or more in a wavelength range of 450 to 750 nm.

10. The organic electroluminescent element according to claim 7, wherein the capping layer is a stack of two or more layers composed of different compounds or a mixed layer containing two or more different compounds, and contains the amine compound according to any one of claims 1 to 6.

11. An electronic element having a pair of electrodes and an organic layer sandwiched therebetween, wherein the organic layer contains the amine compound according to any one of claims 1 to 6.

12. An electronic device comprising the electronic element according to claim 11.
